# EUROPEAN PATENT SPECIFICATION

(11) **EP 2 739 652 B1**
(45) Date of publication and mention of the grant of the patent: **05.04.2017**
(21) Application number: 12753083.0
(22) Date of filing: 03.08.2012
(51) Int. Cl.: C07K 16/14

(54) **CAHGT1P INHIBITORS FOR USE IN THE TREATMENT OF CANDIDIASIS**
CAHGT1P-HEMMER ZUR VERWENDUNG BEI DER BEHANDLUNG VON KANDIDIOSE
INHIBITEURS CAHGT1P À UTILISER DANS LE TRAITEMENT DE LA CANDIDOSE

(30) Priority: 04.08.2011 EP 11176599
(43) Date of publication of application: 11.06.2014
(73) Proprietor: Medizinische Universität Innsbruck, 6020 Innsbruck (AT)
(72) Inventor: WÜRZNER, Reinhard, 6410 Telfs (AT); LESIAK-MARKOWICZ, Iwona, 1110 Wien (AT)
(74) Representative: Vossius & Partner Patentanwälte Rechtsanwälte mbB
(86) International application number: PCT/EP2012/065267
(87) International publication number: WO 2013/017691

(56) References cited:
- LESIAK I ET AL: "Candida albicans HGT1 is a multifunctional complement evasion molecule", MOLECULAR IMMUNOLOGY, PERGAMON, GB, vol. 46, no. 14, 1 September 2009 (2009-09-01), page 2835, XP026438429, ISSN: 0161-5890 [retrieved on 2009-08-06]
- RICKLIN DANIEL ET AL: "Complement-targeted therapeutics", NATURE BIOTECHNOLOGY, NATURE PUBLISHING GROUP, NEW YORK, NY, US, vol. 25, no. 11, 1 November 2007 (2007-11-01), pages 1265-1275, XP002546813, ISSN: 1087-0156, DOI: 10.1038/NBT1342 [retrieved on 2007-11-07]
- KRAICZY P ET AL: "Complement escape of human pathogenic bacteria by acquisition of complement regulators", MOLECULAR IMMUNOLOGY, PERGAMON, GB, vol. 43, no. 1-2, 1 January 2006 (2006-01-01), pages 31-44, XP025037093, ISSN: 0161-5890, DOI: 10.1016/J.MOLIMM.2005.06.016 [retrieved on 2006-01-01]
- DATABASE UniProt [Online] 1 November 1998 (1998-11-01), "RecName: Full=High-affinity glucose transporter;", XP002666557, retrieved from EBI accession no. UNIPROT:O74713 Database accession no. O74713 & VARMA A ET AL: "MOLECULAR CLONING AND FUNCTIONAL CHARACTERISATION OF A GLUCOSE TRANSPORTER, CAHGT1, OF CANDIDA ALBICANS", FEMS MICROBIOLOGY LETTERS, BLACKWELL PUBLISHING, AMSTERDAM, NL, vol. 182, no. 1, 1 January 2000 (2000-01-01), pages 15-21, XP001088876, ISSN: 0378-1097, DOI: 10.1016/S0378-1097(99)00558-3
- VOGL ET AL: "Immune evasion by acquisition of complement inhibitors: The mould Aspergillus binds both factor H and C4b binding protein", MOLECULAR IMMUNOLOGY, PERGAMON, GB, vol. 45, no. 5, 25 October 2007 (2007-10-25), pages 1485-1493, XP022402182, ISSN: 0161-5890, DOI: 10.1016/J.MOLIMM.2007.08.011
- LESIAK I ET AL: "Candida albicans CaHGT1 and its role in complement inhibition and modulation of other virulence factors", INTERNATIONAL JOURNAL OF MEDICAL MICROBIOLOGY; 60TH ANNUAL MEETING OF THE DEUTSCHEN-GESELLSCHAFT-FUR-HYGIENE-UND-MIK ROBIOLOGIE, URBAN UND FISCHER, DE; DRESDEN, GERMANY, vol. 298, no. Suppl. 2 (45), 5 September 2008 (2008-09-05), page 23, XP009155172, ISSN: 1438-4221, DOI: 10.1016/J.IJMM.2008.08.001 [retrieved on 2008-08-26]
- LESIAK-MARKOWICZ I ET AL: "The Candida albicans factor H binding molecule Hgt1p also functions as complement receptor 3 (CR3) and HIV binding molecule", INTERNATIONAL JOURNAL OF MEDICAL MICROBIOLOGY; 63RD ANNUAL MEETING OF THE GERMAN-SOCIETY-FOR-HYGIENE-AND-MICROBIOLOG Y, URBAN UND FISCHER, DE; ESSEN, GERMANY, vol. 301, no. Suppl. 1 (47), 1 September 2011 (2011-09-01), page 10, XP009155169, ISSN: 1438-4221, DOI: 10.1016/J.IJMM.2011.08.002 [retrieved on 2011-08-26]
- LESIAK-MARKOWICZ IWONA ET AL: "Candida albicans Hgt1p, a multifunctional evasion molecule: complement inhibitor, CR3 analogue, and human immunodeficiency virus-binding molecule.", THE JOURNAL OF INFECTIOUS DISEASES 1 SEP 2011 LNKD- PUBMED:21844307, vol. 204, no. 5, 1 September 2011 (2011-09-01), pages 802-809, XP009155168, ISSN: 1537-6613

## Description

The present invention is in the field of medical microbiology, in particular mycology, and relates to the treatment of fungal infections such as candidiasis by masking the glucose transporter CaHgt1p of *Candida albicans* or functional equivalents thereof.

### Background of the invention

*Candida* belongs to the genus of yeasts. Some members of the *Candida* genus can be found on normal skin of adult humans as well as in the normal flora of the mucous membranes of the respiratory, gastrointestinal and genital tracts. However, particularly *Candida albicans* may also cause superficial and systemic infections known as "candidiasis".

*Candida albicans* is a normal colonizer of mucous membranes, predominantly known as physiological inhabitant of the human gastrointestinal tract. In recent years *Candida* has emerged as a leading pathogen in the opportunistic fungal infection of locally or systemically immunocompromised hosts (Whiteway M, Bachewich C. Morphogenesis in Candida albicans. Ann Rev Microbiol 2007; 61:529-53). Pathogenicity of *C*. *albicans* is mediated by various virulence factors, including surface-expressed or secreted proteins, such as adhesins, which allow adherence to epithelial and endothelial cells, or aspartyl proteases and phospholipases. These important factors enable *C. albicans* dissemination in the host, enhancing its ability to colonize organs (Munro CA, Hube B. Anti-fungal therapy at the HAART of viral therapy. Trends Microbiol 2002; 10:177-8). Additionally, the reversible transition between unicellular yeast cells and filamentous growth forms is considered essential for the infection process. Increased adhesion to host cells also facilitates tissue penetration after the expression of specific surface antigens during hyphal growth (Whiteway et al., loc. cit.). Early diagnosis of invasive fungal infections is difficult and treatment problematic due to a limited number of clinically available drugs (Richardson M, Lass-Flor1 C. Changing epidemiology of systemic fungal infections. Clin Microbiol Infect 2008; 14:5-24). A detailed knowledge regarding interactions between fungi and host immunity is, therefore, important for recognition and treatment of infections.

The innate immunity plays a crucial role in antifungal defence, via immediate reactions and recognition of a broad spectrum of microbial pathogens. The complement system is a key machinery of the innate immunity, recognising and killing pathogens such as bacteria, virus infected cells, parasites as well as fungi (Speth C, Rambach G, Würzner R, Lass-Flor1 C. Complement and fungal pathogens: an update. Mycoses 2008; 51:477-96). Complement components are activated within seconds or minutes after entry of microorganisms into the host. The cascade of sequential activation steps of complement factors leads to the opsonisation, chemotaxis and also to the formation of the terminal complement complex, termed membrane attack complex when formed on a membrane, destroying membranes of pathogens and causing osmotic lysis. In the case of fungal pathogens, opsonisation may play an important role in phagocytosis (Speth et al., loc. cit.).

The complement cascade is tightly regulated - also to preserve normal "self' cells - by several protein components, which are present in soluble form in different body fluids or bound to cell membranes. Factor H (FH), a soluble plasma protein, is the main fluid phase regulator of the complement alternative pathway (Józsi M, Zipfel PF. Factor H family proteins and human diseases. Trends Immunol 2008; 29:380-7). It is employed by various pathogens for their protection against complement destruction when circulating in the host (Kraiczy P, Würzner R. Complement escape of human pathogenic bacteria by acquisition of complement regulators. Mol Immunol 2006; 43:31-44). Further, C4b-binding protein (C4BP), the main fluid-phase inhibitor of the classical and lectin complement pathways, can also decorate the surface of various human pathogens, conferring resistance to complement destruction and thus promoting fungal pathogenicity (Speth et al., loc. cit.). *Candida* spp. (Meri T, Hartmann A, Lenk D, Eck R, Würzner R, Hellwage J, Meri S, Zipfel PF. The yeast Candida albicans binds complement regulators factor H and FHL-1. Infect Immun 2002; 70:5185-92; Meri T, Blom AM, Hartmann A, Lenk D, Meri S, Zipfel PF. The hyphal and yeast forms of Candida albicans bind the complement regulator C4b-binding protein. Infect Immun 2004; 72:6633-41), similar to moulds such as *Aspergillus* spp. (Vogl G, Lesiak I, Jensen DB, Perkhofer S, Eck R, Speth C, Lass-Flor1 C, Zipfel PF, Blom AM, Dierich MP, Würzner R. Immune evasion by acquisition of complement inhibitors: the mould Aspergillus binds both factor H and C4b binding protein. Mol Immunol 2008; 45:1485-93), can bind factor H as well as C4BP. The glucose transporter HGT1 from *Candida albicans* (CaHGT1) is a complement evasion molecule and binds to C4BP and FH (Lesiak et al., Molecular Immunology 46 (2009), p. 2835)).

Candidiasis is commonly treated with antimycotics. Commonly used antifungal drugs for the treatment of candidiasis include topical clotrimazole, topical nystatin, fluconazole and topical ketoconazole. However, drug resistance has been reported for certain populations of *Candida albicans* (Cowen LE, Nantel A, Whiteway MS, Thomas DY, Tessier DC, Kohn, LM, Anderson JB. Population genomics of drug resistance in Candida albicans. Proc Nat Acad Sci 2002; 99(14):9284-9). Moreover, the lethality of systemic candidiasis in HIV patients is more than 50 % even under therapy.

Hence, there is a need for novel therapeutic strategies and drugs for the treatment of fungal infections such as candidiasis and aspergillosis and infections with *Candida albicans* in particular.

### Summary of the invention

The present invention is based on the surprising finding of the inventors that the glucose transporter HGT1 from *Candida albicans* (herein also termed *CaHGT1* and CaHgtlp) is involved in evasion of phagocyctosis. In particular, the inventors have found that CaHgt1p may act as complement receptor 3 (CR3) analogue. CR3 is a human cell surface receptor found on polymorphonuclear leukocytes, particularly neutrophils, and binds to C3b, a regulatory cleavage product of complement component 3 (C3).

The present invention is directed to the inhibition or masking of CaHgtlp, thereby overcoming evasion of phagocytosis by *C. albicans.*

In one aspect the present invention relates to molecules capable of masking, antagonizing, inhibiting or blocking CaHgt1p. In particular the invention relates to a molecule able of masking ('masking molecule') CaHgt1p to prevent binding to complement component C3b. More in particular the present invention relates to a masking antibody, which inhibits binding between CaHgtlp and C3b.

The present invention relates to a CaHgtlp masking antibody, for use in the treatment of candidiasis in a subject, particularly for use in the treatment of a *Candida albicans* infection in a subject.

Also described are methods for assessing the activity of a candidate molecule suspected of being a masking molecule of the glucose transporter CaHgtlp of *Candida albicans.*

### Description of drawings

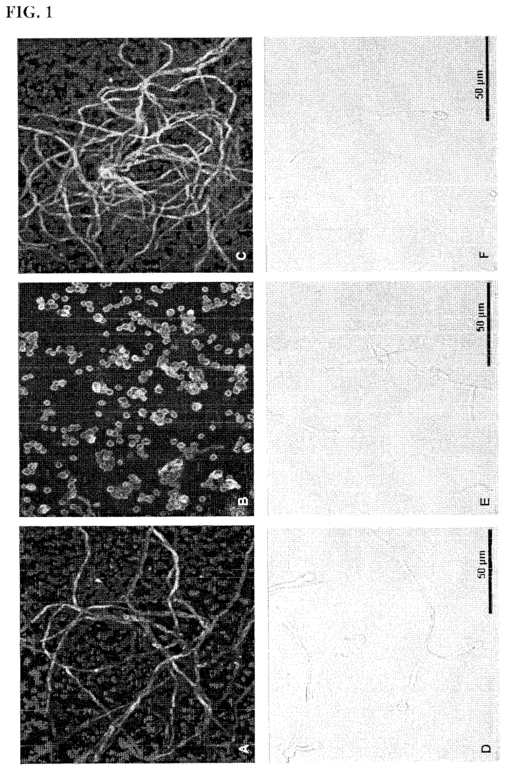

**Figure 1****:** Morphology of *C*. *albicans* hgt1Δ/Δ. Microscopic analyses of cellular morphology, in part via immunofluorescence using anti-Candida IgG (A-C), of C. *albicans* hgt1Δ/Δ (B, E) compared to SN152 parental (A, D) and hgt1Δ/Δ::HGT1 restored (C, F) strains after 24h incubation in RPMI at 30°C (A-C) or at 37°C (D-F). Representative examples of quadruplicate experiments are shown.

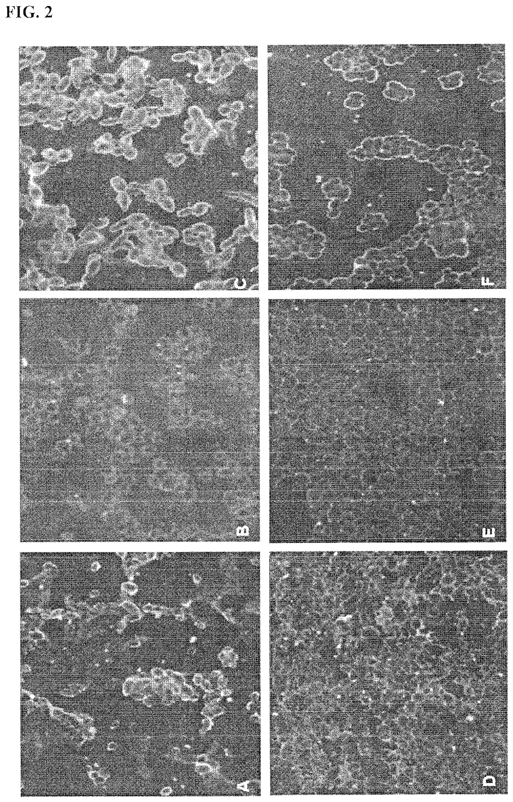

**Figure 2****:** Binding of factor H (FH) by *C. albicans* hgt1Δ/Δ - qualitative assessment. Immunofluorescence analyses of binding of FH from EDTA plasma (A-C) or in its purified form (D-F) by hgt1Δ/Δ (B, E) compared to SN152 parental (A, D) and hgt1Δ/Δ::HGT1 restored (C, F) strains via goat anti-FH IgG. Representative examples of quadruplicates experiments are shown.

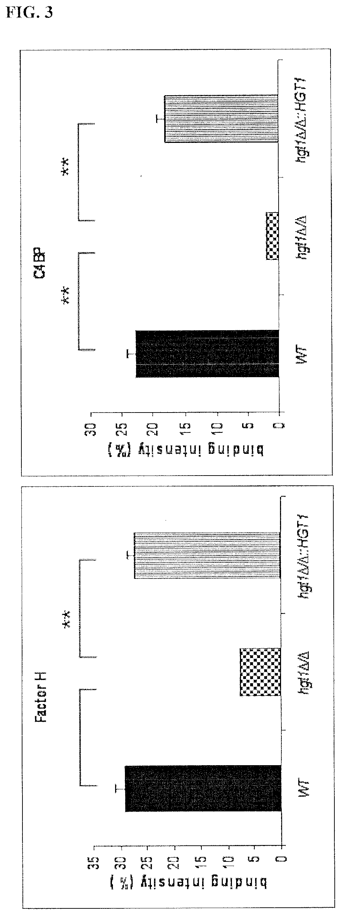

**Figure 3****:** Binding of factor H (FH) and C4 binding protein (C4BP) by *C*. *albicans* hgt1Δ/Δ-quantitative assessment. Binding of FH (A) and C4BP (B) to SN152 parental (or wild type, WT, black columns), hgt1Δ/Δ (chequered columns) and hgt1Δ/Δ::HGT1 restored (striped columns) strains was assessed by determining the percentage of binding cells within the entire population. Results from quadruplicate experiments performed on four different days are shown; **, p<0.01.

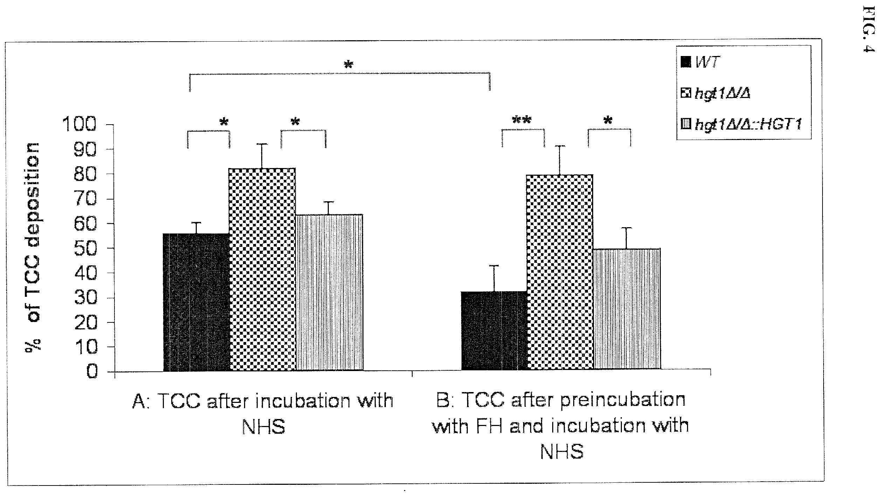

**Figure 4****:** Deposition of terminal complement complex (TCC) on *C. albicans* hgt1Δ/Δ as a measure of complement activation on the yeast cell surface. Deposited TCC was detected by FACS analyses using mouse anti-neoC9 antibodies followed by FITC-labelled anti-mouse antibodies on SN152 parental (black columns), hgt1Δ/Δ (checkered columns), and hgt1Δ/Δ::HGT1 restored (striped columns) strains after incubation with NHS (A) or after preincubation with factor H (50 µg/ml) followed by incubation with NHS (B). Percentages of deposited TCC on the yeast cell surface are shown. Cells incubated in buffer only were used as 100% control. Results from quadruplicate experiments performed on four different days are shown; *, p<0.05, **, p<0.01.

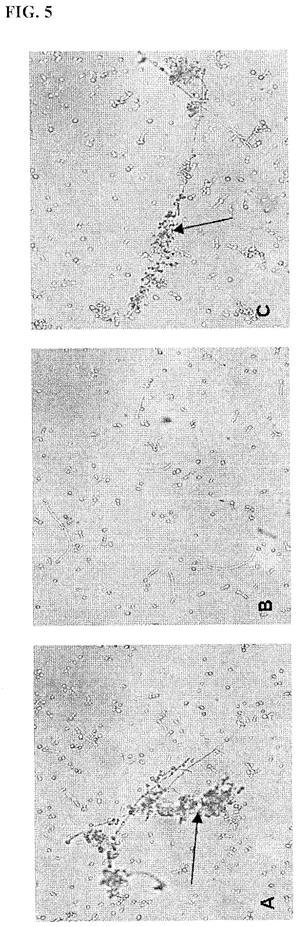

**Figure 5****:** Rosette formation on *C. albicans* hgt1Δ/Δ as a measure of CR3 analogue expression on the yeast cell surface. Rosettes (indicated by arrows) of *C. albicans* SN152 parental and hgt1Δ/Δ::HGT1 restored (C) strains after incubation with complement-coated sheep erythrocytes for 1 h. No rosettes were observed for hgt1Δ/Δ at 1 h (not shown) and not even after extended periods of 12 h (5B) and 24 h. Results from quadruplicate experiments performed on four different days are shown.

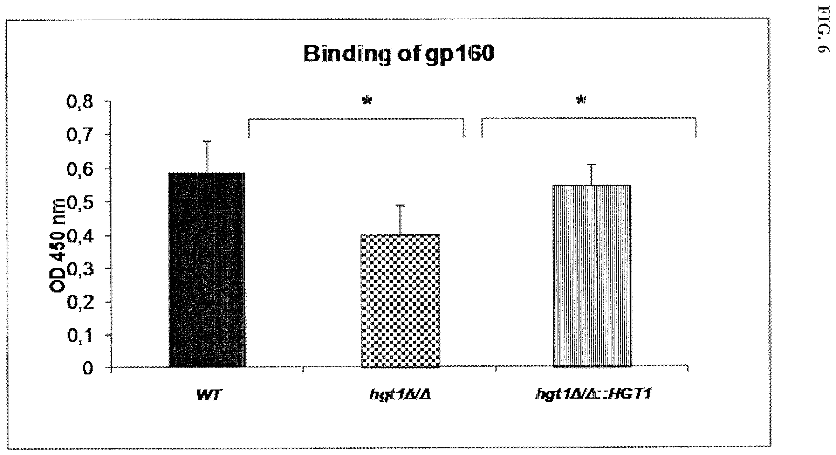

**Figure 6****:** Binding of recombinant HIV gp160 to *C. albicans.* Binding of recombinant HIV gp160 to *C. albicans* SN152 parental (black column), hgt1Δ/Δ (chequered column) and hgt1Δ/Δ::HGT1 restored (striped column) strains was determined by yeast cell ELISA. Human HIV-antibody positive serum (1:500) and anti-human-AP IgG served as detecting reagents. Results from quadruplicate experiments performed on four different days are shown.*, p<0.05.

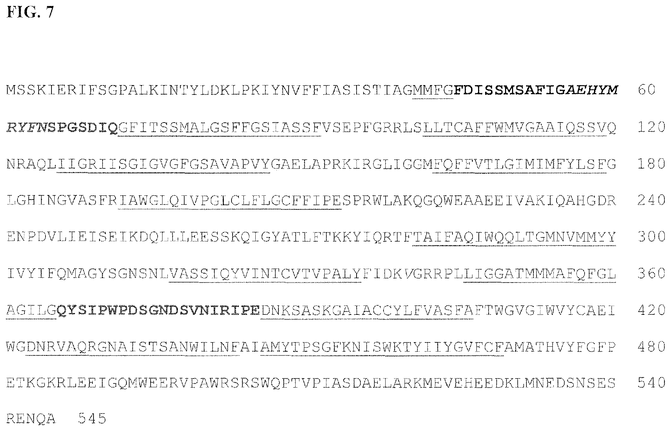

**Figure 7**: Amino acid sequence of CaHgtlp (also see SEQ ID NO: 2). Underlined residues: putative membrane spanning segments; bold residues: residues that belong to preferred epitopes; italic and bold residues: residues that belong to the most preferred epitopes.

### Detailed description of the invention

The present invention relates to a masking antibody against *Candida albicans* glucose transporter HGT1 (CaHgtlp), wherein the antibody is able to inhibit binding of complement component C3b to CaHgtlp.

CaHgtlp (*Candida albicans* high-affinity glucose transporter 1 protein) was the first glucose transporter from *Candida albicans* that has been cloned and characterized (Varma A, Singh BB, Karnani N, Lichtenberg-Fraté H, Höfer M, Magee BB, Prasad R. Molecular cloning and functional characterisation of a glucose transporter, CaHGT1, of Candida albicans. FEMS Microbiol Let 2000; 182:15-21). The DNA sequence (GenBank accession number Y16834) of the *CaHGT1* gene comprises an ORF encoding the 545 amino acid protein CaHgt1p with a predicted molecular mass of 60.67 kDa. The nucleic acid sequence (according to GenBank accession number Y16834) of the gene encoding CaHgtlp is provided in SEQ ID NO: 1 and the corresponding polypeptide sequence is set out in SEQ ID NO: 2. The polypeptide sequence of an isoform of CaHgtlp is provided in SEQ ID NO: 3. The CaHgtlp has 12 putative transmembrane domains (Varma et al., loc. cit.; Fan J, Chaturvedi V, Shen SH. Identification and Phylogenetic Analysis of a Glucose Transporter Gene Family from Human Pathogenic Yeast Candida albicans. J Mol Evol 2002; 55:336-46).

The present invention is based on the surprising finding of the inventors that CaHgtlp is involved in evasion of phagocytosis. In particular, the inventors found that CaHgtlp may function as complement receptor 3 (CR3) analogue. CR3 is a human cell surface receptor found on polymorphonuclear leukocytes, particularly neutrophils, and binds to C3b, a regulatory cleavage product of complement component 3 (C3). C3b serves as an opsonizing agent ultimately leading to phagocytosis. Without being bound by theory, it is believed that CaHgtlp is able to bind to C3b thereby preventing the induction of phagocytosis.

The present invention is directed to the inhibition or masking of CaHgtlp, thereby overcoming evasion of phagocytosis by *C. albicans.* More in particular, the present invention is directed to inhibiting C3b binding to CaHgtlp.

In one aspect the present invention relates to a masking antibody of the glucose transporter CaHgtlp of *Candida albicans* for use in the treatment of fungal infections such as candidiasis in a subject. Also masking molecules, particularly masking antibodies, of functional equivalents and derivatives of CaHgt1p from other species of the Candida genus, the Aspergillus genus and the Zygomycota phylum are described herein. Preferably, the masking antibody masks the extracellular domain or part of CaHgtlp. More preferably, the masking antibody inhibits binding of complement components to the extracellular domain or part of CaHgtlp. According to the invention the masking antibody inhibits binding of complement component C3b to CaHgtlp.

In a preferred embodiment of the present invention the masking antibody of the glucose transporter CaHgt1p of *Candida albicans* is used in the treatment of a fungal infection, particularly of candidiasis, preferably an infection with *Candida albicans* in the subject.

The subject in the context of the present invention may be a human or non-human animal, preferably a mammal and most preferably a human. In a particular embodiment of the invention the subject may also be immunosuppressed or immunocompromised or has a weakened or undeveloped immune system.

The subject in particular embodiments has or is prone to a metabolic illnesses such as diabetes or has or is prone to cachexia or has or is prone to cancer or has undergone or is undergoing cancer therapy such as chemotherapy or radiotherapy or had or is in need of an organ transplantation.

More in particular, the subject may have a primary (e.g. hereditary) or secondary (i.e. acquired) immunodeficiency. In some embodiments the subject may be positive for the human immunodeficiency virus (HIV) ("HIV positive") or may have developed the acquired immunodeficiency syndrome (AIDS).

The candidiasis may for instance be selected from the group of oral candidiasis (thrush), Perlèche (Angular cheilitis), Candidal vulvovaginitis (vaginal yeast infection), Candidal intertrigo, Diaper candidiasis, Congenital cutaneous candidiasis, Perianal candidiasis, Candidal paronychia, Erosio interdigitalis blastomycetica, Chronic mucocutaneous candidiasis, Systemic candidiasis, Candidid and Antibiotic candidiasis.

The subject having candidiasis in the context of the present disclosure has an infection with a species from the Candida genus, preferably selected from *Candida albicans*, *Candida tropicalis*, *Candida parapsilosis, Candida guilliermondi*, *Candida dubliniensis, C. stellatoidea, C. krusei, C. kyfer* and *Candida glabrata.* More preferably the subject has an infection with *Candida albicans* or *Candida dubliniensis.* Most preferably the subject has an infection with *Candida albicans.*

The subject having aspergillosis in the context of the presentdisclosure has an infection with a species from the Aspergillus genus, preferably selected from *Aspergillus fumigatus*, *Aspergillus flavus* and *Aspergillus terreus.*

The subject may for example have a fungal infection, particularly a *Candida albicans* infection, of the oral cavity ("thrush" in case of *Candida albicans*), the pharynx or esophagus, the gastrointestinal tract, the urinary bladder, or the genitalia (vagina, penis). The fungal infection, particularly the *Candida albicans* infection may be an opportunistic infection, especially in the case of HIV/AIDS patients or other patients with a weakened immune system such as subjects that have undergone chemotherapy or that are undergoing chemotherapy e.g. against cancer. In certain particular embodiments of the invention the subject may have a superinfection or a co-infection with another pathogen.

While in principle the masking molecule according to the present disclosure could be any kind of molecule as long as it inhibits complement binding to CaHgtlp and preferably binding of C3b to CaHgtlp, it is particularly preferred that the masking molecule is an antibody or functional fragment thereof.

The masking antibody or functional fragment thereof, according to the present invention preferably binds to epitopes on the extracellular part or domain of CaHgt1p This means that the antibody is preferably specific for epitopes on the extracellular part or domain of CaHgt1p...

As outlined herein above, the present invention in a particular aspect relates to a masking antibody against glucose transporter CaHgtlp of *Candida albicans.* In a particular embodiment the antibody is able to mask the extracellular part of CaHgtlp. More in particular, the antibody is able to inhibit binding of components of the complement system to CaHgtlp. According to the invention, the antibody is able to inhibit binding of complement component C3b to CaHgtlp.

It is therefore preferred that the antibody is specific for an extracellular epitope of CaHgtlp. The epitope may be a linear epitope or a conformational epitope. In a particular embodiment the epitope is a linear epitope comprised in an amino acid sequence selected from SEQ ID NO: 4 and SEQ ID NO: 5. Preferably, the epitope is SEQ ID NO: 6 or is comprised in SEQ ID NO: 6. Other epitopes that might be used are given in table 1. The epitopes of SEQ ID NO: 4-8 might be part of a conformational epitope comprising two or more of the sequences, e.g. the epitopes according to SEQ ID NO: 7 and 8 might be part of a conformational epitope together with one or more of SEQ ID NO: 4-6.

**Table 1: Amino acid sequences of particular epitopes or which comprise preferred epitopes**

| **Sequence** (N-term → C-term) | **SEQ ID NO** |
|---|---|
| FDISSMSAFIGAEHYMRYFNSPGSDIQ | SEQ ID NO: 4 (preferred) |
| QYSIPWPDSGNDSVNIRIPE | SEQ ID NO: 5 (preferred) |
| AEHYMRYFN | SEQ ID NO: 6 (most preferred) |
| IVYIFQMAGYSGNSNL | SEQ ID NO: 7 |
| LGHINGVASFR | SEQ ID NO: 8 |

Hence, in one embodiment the antibody specifically binds to a peptide of an amino acid sequence selected from the group of SEQ ID NO: 4, SEQ ID NO: 5, SEQ ID NO: 6, SEQ ID NO: 7 and SEQ ID NO: 8, preferably selected from SEQ ID NO: 4, SEQ ID NO: 5 and most preferably SEQ ID NO: 6.

The invention also pertains to an antibody obtained by immunisation of a host animal such as a rabbit, mouse, goats or donkey using the antigen CaHgtlp or a fragment thereof such as a peptide having an amino acid sequence selected from the group of SEQ ID NO: 4, SEQ ID NO: 5, SEQ ID NO: 6, SEQ ID NO: 7 and SEQ ID NO: 8, preferably selected from SEQ ID NO: 4, SEQ ID NO: 5 and most preferably SEQ ID NO: 6.

In case of a functional equivalent or derivative of CaHgtlp in the context of the present disclosure, the masking antibody is preferably directed against an extracellular part or domain of the functional equivalent or derivative such that when the antibody binds (i.e. masks) the components of the complement system and particularly C3b cannot bind to the functional equivalent or derivative of CaHgtlp.
For example in case of *Candida dubliniensis*, the masking antibody may specifically bind to a peptide of amino acid sequence SEQ ID NO: 9.

A 'masking molecule' (or particularly a 'masking antibody') herein refers to a molecule (or particularly an antibody) that is able to prevent binding of a normal ligand to CaHgt1p (or functional equivalent or derivative from other species), e.g. by inhibiting binding of that ligand, particularly by sterically blocking the binding site for that ligand. In the context of the present invention the ligand is preferably a component of the complement system that belongs to a pathway ultimately leading to complement induced phagocytosis. According to the invention, the ligand is C3b.

In context of the present invention, the term "antibody" or "antibody molecule" relates to full immunoglobulin molecules, preferably IgMs, IgDs, IgEs, IgAs or IgGs, more preferably IgG1, IgG2, IgG2b, IgG3 or IgG4 as well as to parts of such immunoglobulin molecules. Furthermore, the term relates to modified and/or altered antibody molecules, like chimeric and bovinized or humanized antibodies. In a preferred embodiment, the antibody is humanized. The term also relates to monoclonal or polyclonal antibodies as well as to recombinantly or synthetically generated/synthesized antibodies. In a preferred embodiment, the anti-CaHgtlp antibody is a monoclonal antibody. The term also relates to intact antibodies as well as to antibody fragments thereof, like, separated light and heavy chains, Fab, Fab/c, Fv, Fab', F(ab')₂. The terms "antibody" and "antibody molecule" are herein used interchangeably and also comprise bifunctional antibodies and antibody constructs, like single chain Fvs (scFv) or antibody-fusion proteins. Also a single-chain antibody, a chimeric antibody, a CDR-grafted antibody, a bivalent antibody-construct, an antibody-fusion protein or a synthetic antibody is within the scope of the invention. Further details on the term "antibody molecule" of the invention are provided herein below.

Techniques for the production of antibodies are well known in the art and described, e.g. in Howard and Bethell (2000), Basic Methods in Antibody Production and Characterization, Crc. Pr. Inc.. Antibodies directed against CaHgt1p can be obtained, e.g., by direct injection of CaHgt1p (or a fragment thereof) into an animal or by administering the polypeptide (or a fragment thereof) to an animal. The antibody so obtained will then bind CaHgtlp (or a fragment thereof) itself. In this manner, even a fragment of CaHgt1p can be used to generate antibodies binding the whole polypeptide, as long as said binding is "specific" as defined herein below. Preferably, peptides comprising or consisting of the sequences of SEQ ID NO: 4, SEQ ID NO: 5 and SEQ ID NO: 6 are used for the generation of antibodies.

With the normal skill of the person skilled in the art and by routine methods, the person skilled in the art can easily deduce from the CaHgtlp amino acid sequences provided herein relevant epitopes (also functional fragments) of CaHgtlp which are useful in the generation of antibodies like polyclonal and monoclonal antibodies. However, the person skilled in the art is readily in a position to also provide for engineered antibodies like CDR-grafted antibodies or also humanized and fully human antibodies and the like.

Particularly preferred in the context of the present invention are monoclonal antibodies. For the preparation of monoclonal antibodies, any technique which provides antibodies produced by continuous cell line cultures can be used. Examples for such techniques include the hybridoma technique, the trioma technique, the human B-cell hybridoma technique and the EBV-hybridoma technique to produce human monoclonal antibodies (Shepherd and Dean (2000), Monoclonal Antibodies: A Practical Approach, Oxford University Press, Goding and Goding (1996), Monoclonal Antibodies: Principles and Practice - Production and Application of Monoclonal Antibodies in Cell Biology, Biochemistry and Immunology, Academic Pr Inc, USA).

Preferably in the context of the present invention a monoclonal antibody is to be used. In particular antibodies of mammalian origin are useful in context of the invention. As outlined above, such antibodies can be produced by a hybridoma and/or by a host transformed with an expression vector containing genetically engineered antibody genes. The antibody masks CaHgt1p as described herein above.

The antibody derivatives can also be produced by peptidomimetics. Further, techniques described for the production of single chain antibodies (see, inter alia, US Patent 4,946,778) can be adapted to produce single chain antibodies specifically recognizing CaHgtlp. Also, transgenic animals maybe used to express humanized antibodies to CaHgtlp.

The present invention also envisages the production of a specific antibody against CaHgtlp. This production is based, for example, on the immunization of animals, like mice. However, also other animals for the production of antibody/antisera are envisaged within the present invention. For example, monoclonal and polyclonal antibodies can be produced by rabbit, mice, goats, donkeys and the like. A polynucleotide encoding *CaHGT1* can be subcloned into an appropriated vector, wherein the recombinant polypeptide is to be expressed in an organism being able for an expression, for example in bacteria. Thus, the expressed recombinant protein can be intra-peritoneally injected into a mice and the resulting specific antibody can be, for example, obtained from the mice serum being provided by intra-cardiac blood puncture. The present invention also envisages the production of specific antibodies against CaHgtlp by using a DNA vaccine strategy. DNA vaccine strategies are well-known in the art and encompass liposome-mediated delivery, by gene gun or jet injection and intramuscular or intradermal injection. Thus, antibodies directed against CaHgtlp can be obtained by directly immunizing the animal by directly injecting intramuscularly the vector expressing the CaHgtlp. The amount of obtained specific antibody can be quantified using an ELISA, which is also described herein below. Further methods for the production of antibodies are well known in the art, see, e.g. Harlow and Lane, "Antibodies, A Laboratory Manual", CSH Press, Cold Spring Harbor, 1988.

The following relates to the production of antibodies which are to be used in accordance with the present invention.
The hybridoma can be produced using CaHgtlp (or fragment thereof) as an antigen, thus eliciting an immune response preferably in a mammal, fusing the resulting immune cells with known parent cells by a known cell fusion method and screening monoclonal antibody-producing cells by a known screening method.
For example, the anti- CaHgt1p antibody may be produced as follows.

The *CaHGT1* gene may be produced by biotechnological means known in the art. The nucleic acid sequence encoding *CaHGT1* (or fragment thereof) may be inserted into a known expression vector, the vector may be introduced into an appropriate host cell and the thereby produced CaHgt1 is used as an antigen in immunization. Preferably, organisms and, in particular mammals used in the immunization process are compatible with the parent cell for use in cell fusion. Thus, such mammals usually include but are not limited to, rodents such as mice, rats, hamsters and the like.
The immunization procedure with CaHgt1p (or fragment thereof) is carried out using a routine methods. For example, immunization involves the intraperitoneal or subcutaneous administration of CaHgtlp (or fragment thereof) to the mammal. CaHgtlp (or fragment thereof) which has been diluted and suspended in an appropriate amount of phosphate buffered saline (PBS) or physiological saline and the like may be mixed with an appropriate amount of a common adjuvant, for example Freund's complete adjuvant. After being emulsified, the mixture is administered to a mammal preferably several times every 4 to 21 days.
After immunization has been carried out and after the increase of the respective antibody level in the serum has been confirmed, the immune cells are isolated from the mammal and are subjected to cell fusion. Preferably, the immune cells are isolated from the spleen. Mammalian myeloma cells (i.e. parent cells which are subjected to cell fusion with the above-mentioned immune cells) include but are not limited to cell lines such as P3X63Ag8.653 (J. Immunol. (1979) 123: 1548-1550), P3X63Ag8U.1 (Current Topics in Microbiology and Immunology (1978) 81: 1-7), NS-1 (Kohler, G. and Milstein, C. , Eur. J. Immunol. (1976) 6: 511-519), MPC-11 (Margulies, D. H. et al., Cell (1976) 8: 405-415), SP2/0 (Shulman, M. et al., Nature (1978) 276: 269-270), FO (de St. Groth, S. F. et al., J. Immunol. Methods (1980) 35: 1-21), S194 (Trowbridge, I.S., J. Exp. Med. (1978) 148: 313- 323), R210 (Galfre, G. et al., Nature (1979) 277: 131-133) and the like.

A known method describing the above cell fusion is, for example, given in Milstein et al. (Kohler, G. and Milstein, C., Methods Enzymol. (1981) 73: 3-46). The cell fusion may be carried out in the presence of a cell fusion accelerator, such as polyethylene glycol (PEG), Sendai virus (HVJ) and the like. In addition, an adjuvant such as dimethyl sulfoxide, may be added to enhance efficiency of the fusion. The preferred ratio of myeloma cells and the immune cells in fusion is 1 to 10. Exemplary culture media to be used in the above cell fusion include RPMI1640 medium and MEM culture medium and the like. Such media allow the growth of the above myeloma cell lines. Further, a serum supplement such as fetal calf serum (FCS) may be added.
In cell fusion, predetermined amounts of the above immune cells and the myeloma cells are mixed well in the above culture liquid, to which a PEG solution previously heated to about 37 oC, for example a PEG solution with a mean molecular weight of about 1000 to 6000, is added at a concentration of 30 to 60% (w/v) and mixed to obtain the desired fusion cells (hybridomas). In order to remove agents which interfere with the growth of hybridomas, the cell culture may be centrifuged, the supernatant removed and the cells resuspended in appropriate culture liquid. These steps may be repeated several times so as to remove the undesirable component as completely as possible.

The hybridoma can then be selected by cultivation in a standard selection medium, for example, in the HAT culture medium (a culture liquid containing hypoxanthine, aminopterin, and thymidine). Cultivation in said HAT culture medium is continued generally for a period of time which is sufficient to kill cells other than the desired hybridoma (for example, non-fused cells). This selection process lasts generally several days to several weeks. Hybridomas that produce the desired antibody are screened by a known dilution method and the antibody is then monoclonally cloned.
In the alternative to the above described method, a hybridoma can be obtained by *in vitro* methods. For example, human lymphocytes may be sensitized *in vitro* with CaHgtlp (or fragment thereof) or CaHgt1p (or fragment thereof)-presenting cells, and the resulting sensitized B lymphocytes are fused with a human myeloma cell, for example U266, in order to obtain the human antibody specifically binding to the CaHgt1p antigen. Furthermore, a transgenic animal having a repertoire of all human antibody genes is immunized with the antigen or the antigen-presenting cells to obtain the human antibody in the method described above (see International Patent Application WO 93/12227, WO 92/03918, WO 94/02602, WO 94/25585, WO 96/34096 and WO 96/33735).
The thus generated monoclonal antibody-producing hybridomas can be subcultured under known culture conditions or can be stored for a prolonged period of time in liquid nitrogen. Monoclonal antibodies may be obtained as follows from the above hybridoma. For example, hybridomas are cultivated according to routine methods and antibodies are obtained from the supernatant. Alternatively, the hybridoma can be administered to and grown in a mammal compatible with said hybridoma and the antibodies are obtained as ascites. The first method is indicated in cases where high-purity antibodies are to be obtained, whereas the latter method is useful for a large scale production of antibodies.

Also a recombinant antibody can be used in accordance with the invention. Such a recombinant antibody may be produced by cloning the antibody of the hybridoma and integrating the antibody gene into a suitable vector. This vector can then be introduced into a host for producing the recombinant antibody; see, for example, Carl, A.K., Borrebaeck, and James, W. Larrick, Therapeutic Monoclonal Antibodies, published in the United Kingdom by Macmillan Publishers Ltd. 1990).
For example, mRNA encoding the variable (V) region of the antibody is isolated from the hybridoma. The isolation of mRNA is conducted by preparing total RNA using, for example, a known method such as the guanidine ultracentrifuge method (Chirgwin, J.M. et al., Biochemistry (1979) 18, 5294-5299), the AGPC method (Chmczynski, P. et al., (1987) 162, 156-159), and then mRNA is purified from the total RNA using the mRNA Purification kit (manufactured by Pharmacia) and the like. Alternatively, mRNA can be directly prepared using the Quick Prep mRNA Purification Kit (manufactured by Pharmacia).

cDNA of the V region of antibody may be synthesized from the mRNA thus obtained using a reverse transcriptase. cDNA may be synthesized using the AMV Reverse Transcriptase First-strand cDNA Synthesis Kit and the like. Alternatively, for the synthesis and amplification of cDNA, the 5'-Ampli FINDER RACE Kit (manufactured by Clontech) and the 5'-RACE method (Frohman, M.A. et al., Proc. Natl. Acad. Sci. U.S.A. (1988) 85, 8998-9002; Belyavsky, A. et al., Nucleic Acids Res. (1989) 17, 2919-2932) that employs polymerase chain reaction (PCR) may be used. The desired DNA fragment is purified from the PCR product obtained and may be ligated to vector DNA. Moreover, a recombinant vector is constructed therefrom and is then introduced into *E. coli* etc., from which colonies are selected to prepare the desired recombinant vector. The base sequence of the desired DNA may be confirmed by a known method such as the dideoxy method.
Once the DNA encoding the V region of the antibody has been obtained, it may be ligated to DNA encoding the constant region (C region) of the antibody, which is then integrated into an expression vector. Alternatively, the DNA encoding the V region of the antibody may be integrated into an expression vector which already contains DNA encoding the C region of the antibody.

In order to produce the antibody for use in the present invention, the antibody gene is integrated as described below into an expression vector, whereby the antibody gene may then be expressed under the control of a regulatory region, for example an enhancer and/or a promoter. Subsequently, the expression vector may be transformed into a host cell and the antibody can then be expressed therein.
In accordance with the present invention, an artificially altered recombinant antibody such as a chimeric antibody and a humanized antibody can be used in order to lower the likelihood of a potential immune response in the human body to the antibody. Such antibodies can be produced according to known methods.
For example, a chimeric antibody can be obtained by ligating the obtained DNA encoding the V region of antibody to DNA encoding the C region of human antibody, which is then integrated into an expression vector and introduced into a host for production of the antibody (see European Patent Application EP 125023, and International Patent Application WO 96/02576). By this method chimeric antibodies for use in the present invention can be obtained.

Humanized antibodies can be produced by grafting the complementarity determining regions (CDRs) of an antibody of a mammal other than the human, for example a murine antibody, into a human antibody (thereby replacing the original human CDRs). The general recombinant DNA technology for preparation of such antibodies is also known (see European Patent Application EP 125023 and International Patent Application WO 96/02576).
Specifically, a DNA sequence which can be generated to ligate the CDR of a murine antibody to the framework region (FR) of a human antibody is synthesized from several divided oligonucleotides having overlapping by PCR. The thus obtained DNA is ligated to the DNA encoding the C region of a human antibody. Then the DNA is integrated into an expression vector, which is introduced into a host for antibody production (see European Patent Application EP 239400 and International Patent Application WO 92/19759).
In CDR grafting, the complementarity determining region and/or FR may be further adapted to maintain antigen binding specifitiy. For example, amino acids in the framework region of the antibody variable region may be substituted to allow the complementarity determining region to form a functional antigen biding site (Sato, K. et al., Cancer Res. (1993) 53, 851-856).

In the generation of chimeric antibodies or humanized antibodies, the C region of human antibody may be used, such as Cγ, and Cγ1, Cγ2, Cγ3, and Cγ4, and the like. The C region of human antibody may be further modified to improve the stability of antibody or the production thereof.
Chimeric antibodies consist of the variable region of antibody derived from a mammal other than the human, whereas the C region is derived from a human antibody. In contrast, humanized antibodies consist of the complementarity determining region of an antibody derived from a mammal other than the human, whereas the framework region (FR) and the C region of the antibody are derived from a human antibody. Accordingly, the risk that such antibodies elicit an immune response in the human body has been reduced which makes such antibodies particularly useful in context of the present invention.
Antibody genes may be expressed and obtained according to known methods. For example, a promoter, an antibody gene to be expressed, and a poly A signal at 3' downstream thereof can be operably linked and introduced into a vector. A non-limiting examples of the promoter/enhancer are human cytomegalovirus immediate early promoter/enhancer, viral promoters/enhancers such as promoters of retrovirus, polyoma virus, adenovirus, and simian virus 40 (SV40), and promoters/enhancers derived from mammalian cells such as human elongation factor 1α (HEF1α). Expression may be performed according to the method of Mulligan et al. (Nature (1979) 277, 108) when SV40 promoter/enhancer is used, or according to the method of Mizushima et al. (Nucleic Acids Res. (1990) 18, 5322) when HEF1α promoter/enhancer is used.

When the antibody is to be produced in a prokaryotic system (e.g. in *E. coli*), a construct may be used which contains an operably linked promoter, a signal sequence for antibody secretion, an antibody gene to be expressed. Non-limiting examples of a promoter to be used in such a context are lacz promoter and araB promoter. The method according to Ward et al. (Nature (1098) 341, 544-546; FASEB J. (1992) 6, 2422-2427) may be used when lacz promoter is used and the method according to Better et al. (Science (1988) 240, 1041-1043) may be used when araB promoter is used. The pelB signal sequence (Lei, S. P. et al., J. Bacteriol. (1987) 169, 4379) can be used to allow antibody secretion into the periplasm of *E. coli.* The structure of the antibody is to be appropriately refolded prior to its use (see, for example, WO 96/30394) after the antibody has been isolated from the periplasm.

The following origins of replication can for example be used: origins of replication derived from SV40, polyoma virus, adenovirus, bovine papilloma virus (BPV) and the like. Furthermore expression vectors can include selectable markers such as the aminoglycoside transferase (APH) gene, the thymidine kinase (TK) gene, *E. coli* xanthine guaninephosphoribosyl transferase (Ecogpt) gene, the dihydrofolate reductase (dhfr) gene and the like for the amplification of the gene copy number in the host cell system.
The antibody may be prepared in *in vitro* or in vivo systems, for example using eukaryotic cells or prokaryotic cells. Exemplary eukaryotic cells to be used are animal cells, plant cells, and fungal cells. Non-limiting examples of animal cells are (1) mammalian cells such as CHO cells, COS cells, myeloma cells, baby hamster kidney (BHK) cells, HeLa cells, and Vero cells, (2) amphibian cells such as Xenopus oocytes, or (3) insect cells such as sf9, sf21, and Tn5. Known plant cells include, for example, those derived from *Nicotiana tabacum,* which may be subjected to callus culture. Known fungal cells include yeasts such as the genus Saccharomyces, more specifically *Saccharomyces cerevisiae,* or filamentous fungi such as the genus Aspergillus, more specifically *Aspergillus niger.* Exemplary prokaryotic cells are bacterial cells such as *Escherichia coli* (*E.coli*), *Bacillus subtilis* and the like. The antibody can be obtained by introducing antibody genes into these cells and culturing the transformed cells in vitro. Exemplary culture liquid to be used include DMEM, MEM, RPMI1640, and IMDM, optionally supplemented with serum supplements such as fetal calf serum (FCS). The antibodies may also be produced in in vivo systems, e.g. in animals (for example mammals or insects) or plants. Non-limiting examples of animals to be used in this context are mammals, goats, pigs, sheep, mice, and cattle (Vicki Glaser, SPECTRUM Biotechnology Applications, 1993). An exemplary insect that may be used is silkworm. For example, antibodies may be produced in vivo by implanting cells into which the antibody gene has been introduced into the abdominal cavity of an animal and the like. A non-limiting example of a plant that may be used in the production of antibodies is tobacco.
Antibody genes are introduced into these animals or plants, and the antibodies are produced in such animals or plants, and recovered. For example, an antibody gene is inserted into the middle of the gene encoding protein which is inherently produced in the milk such as goat β casein to prepare fusion genes. DNA fragments containing the fusion gene into which the antibody gene has been inserted are injected into a goat embryo, and the embryo is introduced into a female goat. The desired antibody is obtained from the milk produced by the transgenic goat born to the goat who received the embryo or offsprings thereof. In order to increase the amount of milk containing the desired antibody produced by the transgenic goat, hormones may be given to the transgenic goat as appropriate. (Ebert, K.M. et al., Bio/Technology (1994) 12, 699-702).
When silkworms are used, baculovirus into which the desired antibody gene has been inserted is infected to the silkworm, and the desired antibody can be obtained from the body fluid of the silkworm (Susumu, M. et al., Nature (1985) 315, 592-594). Moreover, when tabacco is used, the desired antibody gene is inserted into an expression vector for plants, for example pMON 530, and then the vector is introduced into a bacterium such as *Agrobacterium tumefaciens.* The bacterium is then infected to tabacco such as Nicotianatabacum to obtain the desired antibody from the leaves of the tabacco (Julian, K. -C. Ma et al., Eur. J. Immunol. (1994) 24, 131-138).
When antibody is produced in *in vitro* or *in vivo* production systems, as described above, DNA encoding the heavy chain (H chain) or the light chain (L chain) of antibody may be separately integrated into an expression vector and the hosts are transformed simultaneously, or DNA encoding the H chain and the L chain may be integrated into a single expression vector and the host is transformed therewith (see International Patent Application WO 94-11523).

As mentioned, also fragments of antibodies can be used in context of the present invention, such as Fab, F(ab')2, Fv or (bispecific) single-chain Fv (scFv), for example scFv in which Fv's of H chain and L chain were ligated via a suitable linker. Antibodies may be treated with an enzyme, for example, papain or pepsin, to produce antibody fragments. In the alternative, genes encoding such antibody fragments may be constructed, and then introduced into an expression vector, which is expressed in a suitable host cell (see, for example, Co, M. S. et al., J. Immunol. (1994) 152, 2968- 2976; Better, M. and Horwitz, A.H., Methods in Enzymology (1989) 178, 476-496, Academic Press, Inc.; Plueckthun, A. and Skerra, A. , Methods in Enzymology (1989) 178, 476-496, Academic Press, Inc. ; Lamoyi, E., Methods in Enzymology (1986) 121, 652-663; Rousseaux, J. et al., Methods in Enzymology (1986) 121, 663-669; Bird, R. E. et al., TIBTECH (1991) 9,132-137).
scFv can be obtained by ligating the V region of H chain and the V region of L chain of antibody. In the scFv, the V region of H chain and the V region of L chain are preferably ligated via a linker, preferably a peptide linker (Huston, J.S. et al., Proc. Natl. Acad. Sci. U. S.A. (1988) 85, 5879-5883). The V region of H chain and the V region of L chain in the scFv may be derived from any of the above- mentioned antibodies. As the peptide linker for ligating the V regions, any single-chain peptide comprising, for example, 12 - 19 amino acid residues may be used.
DNA encoding scFv can be obtained using DNA encoding the H chain or the H chain V region of the above antibody and DNA encoding the L chain or the L chain V region of the above antibody as the template by amplifying the portion of the DNA encoding the desired amino acid sequence among the above sequences by the PCR technique with the primer pair specifying the both ends thereof, and by further amplifying the combination of DNA encoding the peptide linker portion and the primer pair which defines that both ends of said DNA be ligated to the H chain and the L chain, respectively.

Once DNA encoding scFv is constructed, an expression vector containing it and a host transformed with said expression vector can be obtained by conventional methods, and scFv can be obtained using the resultant host by conventional methods. These antibody fragments can be produced by obtaining the gene thereof in a similar manner to that mentioned above and by allowing it to be expressed in a host. Also modified antibodies, i.e. antibodies associated with various molecules such as polyethylene glycol (PEG) can be used. These modified antibodies can be obtained by chemically modifying the antibodies by known methods.

Antibodies produced and expressed as described above can be separated from the inside or outside of the host cell and may be purified to homogeneity. Separation and purification of the antibody for use in the present invention may be accomplished by affinity chromatograph, e.g. using Protein A column or Protein G column. Examples of carriers used in the Protein A column are Hyper D, POROS, Sepharose F. F. and the like. Alternatively, methods for separation and purification conventionally used for proteins can be used. Separation and purification of the antibody for use in the present invention may be accomplished by combining, as appropriate, chromatography other than the above-mentioned affinity chromatography, filtration, ultrafiltration, salting-out, dialysis and the like. Chromatography includes, for example, ion exchange chromatography, hydrophobic chromatography, gel-filtration and the like. These chromatographies can be applied to HPLC. Alternatively, reverse-phase chromatography can be used.
The concentration of antibody can be determined by measurement of absorbance or by enzyme- linked immunosorbent assay (ELISA) and the like. When absorbance measurement is employed, the antibody for use in the present invention or a sample containing the antibody may be appropriately diluted with PBS and the absorbance measured at 280 nm, followed by calculation using the absorption coefficient of 1.35 OD at 1 mg/ml. When ELISA is used, measurement may conducted as follows. Goat anti- human IgG (manufactured by TAGO) is diluted to 1 µg/ml in 0.1 M bicarbonate buffer, pH 9.6, is added to a 96-well plate (manufactured by Nunc), and is incubated overnight at 4°C to immobilize the antibody.
After blocking, 100 µl of appropriately diluted antibody for use in the present invention or a sample containing the antibody, or 100 µl of human IgG (manufactured by CAPPEL) (the standard) is added, and incubated at room temperature for 1 hour. After washing, 100 ul of 5000-fold diluted alkaline phosphatase-labeled anti-human IgG antibody (manufactured by BIO SOURCE) is added, and incubated at room temperature for 1 hour. After washing, the substrate solution is added and incubated, followed by the measurement of absorbance at 405 nm using the MICROPLATE READER Model 3550 (manufactured by Bio-Rad) to calculate the concentration of the antibody.

The term "CDR" as employed herein relates to "complementary determining region", which is well known in the art. The CDRs are parts of immunoglobulins and T cell receptors that determine the specificity of said molecules and make contact with specific ligand. The CDRs are the most variable part of the molecule and contribute to the diversity of these molecules. There are three CDR regions CDR1, CDR2 and CDR3 in each V domain. CDR-H depicts a CDR region of a variable heavy chain and CDR-L relates to a CDR region of a variable light chain. H means the variable heavy chain and L means the variable light chain. The CDR regions of an Ig-derived region may be determined as described in Kabat (1991). Sequences of Proteins of Immunological Interest, 5th edit., NIH Publication no. 91-3242 U.S. Department of Health and Human Services, Chothia (1987). J. Mol. Biol. 196, 901-917 and Chothia (1989) Nature, 342, 877-883.
In accordance with this invention, a framework region relates to a region in the V domain (VH or VL domain) of immunoglobulins and T-cell receptors that provides a protein scaffold for the hypervariable complementarity determining regions (CDRs) that make contact with the antigen. In each V domain, there are four framework regions designated FR1, FR2, FR3 and FR4. Framework 1 encompasses the region from the N-terminus of the V domain until the beginning of CDR1, framework 2 relates to the region between CDR1 and CDR2, framework 3 encompasses the region between CDR2 and CDR3 and framework 4 means the region from the end of CDR3 until the C-terminus of the V domain; see, inter alia, Janeway, Immunobiology, Garland Publishing, 2001, 5th ed. Thus, the framework regions encompass all the regions outside the CDR regions in VH or VL domains. Furthermore, the term "transition sequence between a framework and a CDR region" relates to a direct junction between the framework and CDR region. In particular, the term "transition sequence between a framework and a CDR region" means the sequence directly located N- and C-terminally of the CDR regions or amino acids surrounding CDR regions. Accordingly, frameworks may also comprise sequences between different CDR regions. The person skilled in the art is readily in a position to deduce from a given sequence the framework regions, the CDRs as well as the corresponding transition sequences; see Kabat (1991) Sequences of Proteins of Immunological Interest, 5th edit., NIH Publication no. 91-3242 U.S. Department of Health and Human Services, Chothia (1987). J. Mol. Biol. 196, 901-917 and Chothia (1989) Nature, 342, 877-883.

The terms "specific" or "specifically recognizing" in the context of antibodies mean in accordance with this invention that the antibody molecule is capable of specifically interacting with and/or binding to at least two amino acids of CaHgt1p as defined herein. Antibodies can recognize, interact and/or bind to different epitopes on the same target molecule. The term "specifically recognizing" may, therefore, also relate to the specificity of the antibody molecule, i.e. to its ability to discriminate between the specific regions of CaHgt1p.

Preferably, the antibody to be used in accordance with the invention does not cross-react with (poly)peptides of similar structure (e.g. further members of the CaHGT family). Cross-reactivity of a panel of constructs under investigation may be tested, for example, by assessing binding of said panel of antibodies under conventional conditions (see, e.g., Harlow and Lane, Antibodies: A Laboratory Manual, Cold Spring Harbor Laboratory Press, 1988 and Using Antibodies: A Laboratory Manual, Cold Spring Harbor Laboratory Press, 1999) to CaHgt1p as well as to a number of more or less (structurally and/or functionally) closely related (poly)peptides. An exemplary polypeptide that is more structurally and/or functionally related is a further CaHGT protein such as CaHGT2 to CaHGT20. Those constructs (i.e. antibodies, (bispecific) scFvs and the like) that bind to CaHgtlp but do not or do not essentially bind to any of the other (poly)peptides mentioned above are considered to be specific for CaHgt1p and may preferably be selected for further studies in accordance with the invention. However, in some very particular embodiments the antibody may not only recognize CaHgt1p but also other members of the CaHGT family.

The term "specifically recognizing" relates not only to a linear epitope but may also relate to a conformational epitope, a structural epitope or a discontinuous epitope consisting of two regions of CaHgt1p or parts thereof. In context of this invention, a conformational epitope is defined by two or more discrete amino acid sequences separated in the primary sequence which come together on the surface of the molecule when the polypeptide folds to the native protein (Sela, (1969) Science 166, 1365 and Laver, (1990) Cell 61, 553-6). For example, the extracellular part or domain of the herein described CaHgtlp may be considered as a "conformational epitope".
The term "discontinuous epitope" means in context of the invention non-linear epitopes that are assembled from residues from distant portions of the polypeptide chain. These residues come together on the surface when the polypeptide chain folds into a three-dimensional structure to constitute a conformational/structural epitope.
The antibodies of the present invention are also envisaged to specifically bind to/interact with a conformational/structural epitope(s) composed of and/or comprising the two regions of CaHgtlp described herein or parts thereof as disclosed herein below.
Accordingly, specificity can be determined experimentally by methods known in the art and methods as disclosed and described herein. Such methods comprise, but are not limited to Western blots, ELISA-, RIA-, ECL-, IRMA-tests and peptide scans.

Inventive antibody molecules can easily be produced in sufficient quantities, inter alia, by recombinant methods known in the art, see, e.g. Bentley, Hybridoma 17 (1998), 559-567; Racher, Appl. Microbiol. Biotechnol. 40 (1994), 851-856; Samuelsson, Eur. J. Immunol. 26 (1996), 3029-3034.

The invention also pertains to the Ca HGT1-antibodies obtained or obtainable by the methods described herein above.

As already mentioned above, the masking molecules, particularly the masking antibodies, according to the invention may be used for the treatment of fungal infections, particularly candidiasis and more particularly infections with *Candida albicans* in a subject. Hence, the present invention in one aspect also pertains to a method of treating a subject having a fungal infection, particularly an infection with *Candida albicans* or having candidiasis by administering a pharmaceutically acceptable amount of the masking molecule, particularly the masking antibody, to said subject. The masking molecule, particularly the masking antibody, may be comprised in a pharmaceutical composition, optionally comprising one or more additional pharmaceutically acceptable excipients, e.g. a pharmaceutically acceptable carrier and/or diluent. Hence, the invention also pertains to such a pharmaceutical composition comprising the masking molecule, particularly the masking antibody, according to the invention and optionally one or more additional excipients.
The invention also relates to the use of the masking molecules, particularly the masking antibodies for the preparation of a medicament for the treatment of a fungal infection, particularly an infection with *Candida albicans* or candidiasis.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., subcutaneously, intramammary and/or intramuscularly. The compositions of the invention may also be administered directly to the target site. The dosage regimen will be determined by the attending physician and clinical factors. As is well known in the medical arts, dosages for any one patient depends upon many factors, including the patient's or subject's size, body surface area, age, the particular compound to be administered, sex, time and route of administration, general health, and other drugs being administered concurrently. Proteinaceous pharmaceutically active matter may preferably be present in amounts between 1 µg and 10 mg/kg body weight per dose; however, doses below or above this exemplary range are envisioned, especially considering the aforementioned factors. If the regimen is a continuous infusion, it should preferably also be in the range of 1 µg to 10 mg units per kilogram of body weight per minute.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the composition of the invention may comprise further agents depending on the intended use of the composition. Said agents may be, e.g., Tween, EDTA, Citrate, Sucrose as well as other agents being suitable for the intended use of the composition that are well-known to the person skilled in the art.

The masking molecule, preferably the masking antibodies, of the present invention may also be co-administered with antifungal drugs (antimycotics) commonly used to treat candidiasis such as but not limited to clotrimazole, nystatin, fluconazole or ketoconazole.

The masking molecules, preferably the masking antibodies, and the pharmaceutical compositions according to the invention are most effective when they are administered before the onset of clinical symptoms in a patient being at risk or being prone to a fungal infection, particularly candidiasis or aspergillosis or zygomycosis, preferably an infection with *Candida albicans.* In a particular embodiment the masking molecules, preferably the masking antibodies, or the pharmaceutical compositions are administered before the onset of a Candida-related sepsis. Patients at risk of acquiring the fungal infections are for example patients that are being monitored, e.g. by PCR-based assays, and in which subsequently a fungal pathogen has been detected even before these patients show clinically apparent symptoms.

The invention also provides for a nucleic acid molecule encoding an inventive antibody molecule as defined herein. Preferably, the nucleic acid molecule of the invention is part of a vector. The present invention therefore also relates to a vector comprising the nucleic acid molecule of the present invention. The vector of the present invention may be, e.g., a plasmid, cosmid, virus, bacteriophage or another vector used e.g. conventionally in genetic engineering, and may comprise further genes such as marker genes which allow for the selection of said vector in a suitable host cell and under suitable conditions. Furthermore, the vector of the present invention may, in addition to the nucleic acid sequences of the invention, comprise expression control elements, allowing proper expression of the coding regions in suitable hosts. Such control elements are known to the artisan and may include a promoter, a splice cassette, translation initiation codon, translation and insertion site for introducing an insert into the vector. Preferably, the nucleic acid molecule of the invention is operatively linked to said expression control sequences allowing expression in eukaryotic or prokaryotic cells.
The present invention also relates to a host cell transfected or transformed with the vector of the invention or a non-human host carrying the vector of the present invention, i.e. to a host cell or host which is genetically modified with a nucleic acid molecule according to the invention or with a vector comprising such a nucleic acid molecule.

Herein disclosed is also a method for assessing the activity of a candidate molecule suspected of being an antagonist, particularly a masking molecule, of the glucose transporter CaHgtlp of *Candida albicans* comprising the steps of:
(a) contacting *Candida albicans* with complement component C3b in the presence of said candidate molecule,
(b) detecting the binding of C3b to the *Candida albicans* cell and comparing it to the binding of C3b to *Candida albicans* in the absence of said candidate molecule,
wherein a decreased binding in the presence of the candidate molecule as compared to the absence of the candidate molecule is indicative for the capacity of the candidate molecule to antagonize CaHgtlp.

Also disclosed is a method for assessing the activity of a candidate molecule suspected of being an antagonist, particularly a masking molecule, of the glucose transporter CaHgtlp of *Candida albicans* comprising the steps of:
(a) contacting *Candida albicans* in the presence of said candidate molecule with erythrocytes that have been preincubated with complement,
(b) detecting rosette formation and comparing it to rosette formation in the absence of said candidate molecule,
wherein a decreased rosette formation in the presence of the candidate molecule as compared to the absence of the candidate molecule is indicative for the capacity of the candidate molecule to antagonize CaHgt1p.

The above described methods for assessing the activity of a candidate molecule can be transferred to other species of the Candida genus, the Aspergillus genus or the Zygomycota phylum and the respective functional equivalents of CaHgt1p. in these species.

Also disclosed is a method of diagnosing a fungal infection, particularly candidiasis or aspergillosis or zygomycosis, preferably an infection with *Candida albicans.* Such a method comprises the step of detecting the CaHgtlp protein or the CaHGT1 gene or a respective mRNA transcript thereof in a sample of a subject or detecting a functional equivalent or derivative of CaHgtlp or CaHGT1 in the sample of the subject. The detection may be nucleic acid based, e.g. using oligonucleotide primers and/or probes such as PCR-based methods, or it may alternatively be based on immunoassays such as ELISA and the like. The detection of CaHgtlp or CaHGT1 in the sample of the subject is indicative for an infection with *Candida albicans.* The detection of the respective functional equivalent or derivative of CaHgtlp or CaHGT1 of other species of the Candida genus, the Aspergillus genus or the Zygomycota phylum is indicative for an infection with said other species. The sample may for example be a blood sample or a tissue or an organ sample such as a swab or smear sample e.g. from the mouth or the gastrointestinal tract or from the urogenital tract. In case of immunoassays primary antibodies against one or more of the epitopes of SEQ ID NO: 4-8 may be used, most preferably antibodies against the epitope of SEQ ID NO: 6 may be used.

### Examples

### Example 1: CaHgtlp as complement inhibitor

### Methods

### Strains and growth conditions

For the yeast *Candida albicans* which can display different morphological forms [1], may cause systemic disease [2-3] and can bind complement regulators [4-9], two representatives have been used: SN152 [10], which was used both as wild type and as parental strain for the knock out procedure detailed below - this was grown on YPD (Yeast-Peptone-Dextrose, Becton Dickinson) agar and then transferred to YPD or RPMI medium (GIBCO-Invitrogen) for 16 hours at 30°C or 37°C - and SC5314 [7] which confirmed all results of SN152 (data not shown).

### Plasma, antibodies, and proteins

Plasma, obtained from five healthy human donors, was pooled; FH was obtained from Calbiochem, polyclonal goat anti-FH IgG from Quidel, alkaline phosphatise conjugated anti-goat IgG and BCIP substrate from Sigma, WU 13-15, a neoepitopespecific anti-neo C9 for the quantitation of the terminal complement complex (TCC) antibody [11] was generated in house, FITC-conjugated anti-goat, anti-rabbit and anti-mouse IgG from Dako, and rabbit anti-Candida IgG from Biotrend. C4BP and polyclonal rabbit anti-C4BP was kindly provided by Anna Blom, Sweden and rabbit, goat and mouse IgG by the Institute of Immunology, University of Gottingen.

### Identification of the Candida albicans FH binding protein

In order to isolate and sequence the DNA corresponding to the fungal protein(s) which react with FH, a *C*. *albicans* SC5314 expression library was generated using the lysogene gt11 virus and the ZAP-cDNA Gigapack II Cloning Kit, both from Stratagene. The SC5314 strain had been grown in RPMI medium overnight at 37°C prior to its use for the expression library. For the identification of FH binding clones, the membranes containing the colonies were probed with 50 µg/ml FH, followed by incubation with goat anti-FH at a 1:50 dilution, followed by secondary alkaline phosphate conjugated anti-goat IgG (1:500) and detection using BCIP substrate. Positive colonies were identified using the chloroform technique [12] and reused at appropriate dilutions for a second screen in order to gain single plaques for DNA isolation and sequencing [13]. Sequences were identified using BLAST nucleotide sequence similarity search, alignment was performed via blastN (comparing nucleotide sequences and blastX (translated query vs. protein database)). A further check for homologies was performed with a translated query in the SWISSPROT database. Results were regarded as significant when ≤ 0.05.

### Construction of Candida albicans hgt1Δ/Δ deletion mutants

Fusion PCR [10] was performed in order to create a gene disruption cassette for *C. albicans* HGT1 coding sequences using appropriate primers. HGT1 gene disruption fragments were created, employing SN152 as parental strain, using a LEU2 marker in the first round, and a HIS1 marker in the second. Disruption of both alleles was confirmed by colony PCR and Southern blot analyses (data not shown). The SAT1 marker cassette of the plasmid pSFS2A and the fusion PCR strategy [10] were used for the generation of the gene complementation construct for HGT1. The wild type allele of HGT1 was inserted into the genome of hgt1Δ/Δ mutants at the locus of the LEU2 marker. Transformation was performed via electroporation and genomic integration events were verified by PCR analysis.

### Cellular morphology of Candida albicans strains

*C. albicans* SN152 wild type and parental, hgt1Δ/Δ deletion mutant and hgt1Δ/Δ::HGT1 revertant strains were incubated in RPMI or YPD medium for 24h at 30°C or 37°C, in 24-well plates. Cellular morpholog y was evaluated by microscopy.

### Indirect immunofluorescence (IF)

*C. albicans* strains, after over-night incubation in YPD medium at 30°C, were placed at 1 x 106/ml (final concentration) on glass slides for one hour at room temperature and fixed with aceton:methanol (1:1). Then they were incubated with rabbit anti-Candida IgG or rabbit IgG or with human EDTA-plasma (50%) or FH (50 µg/ml) for 4 hours at 4°C on slides coated with 50 µl of poly- L-lysine (Sigma) for 30 minutes. After fixation and blocking at room temperature, PBS or goat anti-FH IgG or goat IgG (both at 50 µg/ml) were added. Detection was performed via secondary FITC-labelled antigoat or anti-rabbit IgG at 1:50 dilutions.

### Flow cytometry

*C. albicans* strains, after over-night incubation in YPD medium at 30°C, were incubated at 2 x 106/ml (final concentration) with human EDTA-plasma (50%), FH (50 µg/ml) or C4BP (50 µg/ml) for 2 hours at 4°C. After blocking with 1% PBS-skim milk for 30 minutes, the primary antibodies (goat anti-FH (50 µg/ml); rabbit anti-C4BP (50 µg/ml); mouse anti-TCC (WU 13-15, 50 µg/ml) or control IgG (50 µg/ml)) were added for 1 hour at 4°C. Secondary FITC-labelled anti-goa t, anti-rabbit or anti-mouse IgG were used at dilutions of 1:50 for 30 minutes. Cells were fixed in PBS supplemented with 1% formalin and 0.1% azide. Labelled cells were examined by fluorescent activated cell sorting (FACS, Becton Dickinson) with forward and sideward scatters and 10,000 events were routinely counted.

### Rosetting test with complement-coated sheep erythrocytes

*C. albicans* strains were tested for their ability to form rosettes with sheep erythrocytes, preincubated with complement (HS-haemolytic system, Virion/Serion) as described in detail elsewhere [14]. Briefly, *C. albicans* cells were grown over-night in RPMI medium at 30°C, incubated (1:1) with HS for 1 h, 12 h, or 24 h at 37°C and evaluated by light microscopy.

### Binding of hgt1Δ/Δ mutants to gp160 HIV envelope protein

*C. albicans* strains were tested for their ability to bind gp160 by ELISA. Over-night Candida cell cultures (in RPMI medium at 30°C) were washed twice with PBS and diluted in PBS to 2.5 x 107 cells/ml; 40µl of the cell suspension were distributed to each well of an ELISA plate and incubated for 1h at 30°C. After two careful washing steps with PBS, the recombinant HIV gp160 antigen (a generous gift from F. Dorner (Immuno AG)) was added at a concentration of 5 µg/well and incubated for 2h at 4°C. Human HIV-antibody positive serum (dilution 1:500, pooled from 5 patients) and alkaline phosphatase-conjugated anti-human immunoglobulin IgG (Sigma) served as detecting reagents. The presence of still coated hyphae was microscopically verified after each washing step. A microdilution plate reader was used for recording the absorbance at 450 nm with a reference wavelength of 630 nm.

### Results

### Identification of CaHgt-1p as FH binding molecule and generation of C. albicans hgt1Δ/Δ mutants

CaHgt1p was identified as a FH binding molecule by probing an expression library with serum, followed by anti-FH antibodies. About 100,000 colonies were initially screened. Positive clones were probed with purified FH, followed by anti-FH antibodies. The sequence corresponding to HGT1 was the by far most frequently found one (11 times), sequences of different hypothetical, DNA-processing, metabolic or ribosomal genes less than three times each. Although the sequence of HGT2 is to 93% homologous to HGT1 [15], it was always (truncated, (646-921 bp sequenced) HGT1 sequence (HGT gene size: 1638 bp [15]) found, and never the sequence of HGT2 or of other members of that family. In order to evaluate the role and importance of HGT1, we constructed a homozygous *C*. *albicans* hgt1Δ/Δ deletion mutant using a disruption marker cassette and a modified fusion PCR protocol that permits a rapid and highly efficient generation of homozygous knock-out mutants [10]. The resulting deletion strains, as well as the restored strain hgt1Δ/Δ::HGT1 in which HGT1 was reintegrated, were analyzed for growth and morphology phenotypes.

### Reduced ability of C. albicans hgt1Δ/Δ to form hyphae in RPMI medium at 30°C and 37°C.

Fluorescent microscopic analyses using rabbit anti-Candida IgG and then anti-rabbit-FITC labelled IgG revealed that hgt1Δ/Δ mutants (Fig. 1B), in contrast to parental (Fig. 1A) or restored strains (Fig. 1C), have impaired ability to form hyphae in RPMI medium at 30°C. After incubation at 37°C, hgt1Δ/Δ mutants were able to form hyphae, however, these were much and visibly shorter (Fig. 1E) than those generated by parental (Fig. 1D) or restored strains (Fig. 1F).

### Reduced binding of factor H (FH) to C. albicans hgt1Δ/Δ

Binding of FH, following incubation in EDTA-plasma or with purified FH, to *C. albicans* parental, hgt1Δ/Δ, and restored strains was tested by immuno-fluorescence assay. Fluorescent microscopy results showed that the binding of FH - in its purified form as well as from human EDTA plasma - to *C*. *albicans* hgt1Δ/Δ mutants (Fig. 2B and 2E, respectively) was reduced when compared to parental (Fig. 2A, 2D) or restored strains (Fig. 2C, 2F).

Reduced binding of FH to *C*. *albicans* hgt1Δ/Δ mutants, when compared to binding to parental or restored strains, was also confirmed by FACS using homogeneous cell populations obtained by filtration of the respective yeast cell sus-pensions. *C. albicans* hgt1Δ/Δ mutants had a decreased ability to bind FH in its purified form (Fig. 3A) when compared to parental or restored strains (Fig. 3A).

### Reduced binding of C4b binding protein (C4BP) to C. albicans hgt1Δ/Δ

Binding of C4BP was tested by FACS using homogeneous cell populations obtained by filtration of cell suspensions of parental, hgt1Δ/Δ and restored strains. Following incubation with purified C4BP *C. albicans* hgt1Δ/Δ mutants had a reduced ability to bind C4BP when compared to parental or restored strains (Fig. 3B).

### Increased deposition of terminal complement complex (TCC) on C. albicans hgt1Δ/Δ as a measure of lower inhibition of complement activation on the yeast cell surface

*C. albicans* hgt1Δ/Δ mutants were tested for the deposition of terminal complement complexes (TCC) following complement activation. Yeast cells were preincubated with FH (50 µg/ml) followed by incubation with normal human serum (NHS) and compared with yeast cells incubated with NHS only. Flow cytometry analyses revealed that TCC deposition on the yeast cell surface (after incubation of yeast cells with NHS for 2 hours at 37°C) was much more pronoun ced for the hgt1Δ/Δ mutant than for the parental and restored strains (Fig. 4A) which still have the binding site for the complement inhibitor FH. Preincubation with FH was done to corroborate these findings. While there was no difference in TCC deposition for the hgt1Δ/Δ mutants after preincubation with FH (50 µg/ml, for 2 hours at 4°C followed by incubation with NHS for 2 hours at 37°C; Fig. 4B), as expected from the absence of the major FH binding molecule, the parental strain showed an even lower deposition of TCC upon preincubation with FH (Fig. 4B).

### Reduced rosette formation on C. albicans hgt1Δ/Δ as a measure of low or absent CR3 analogue expression on the yeast cell surface

Whereas formation of rosettes with complement-coated erythrocytes was observed for parental (Fig. 5A) and restored (Fig. 5C) strains after 1 h, the hgt1Δ/Δ mutant failed to form rosettes both at 1 h (not shown), and even after prolonged incubations for 12 h (Fig. 5B) or 24 h (data not shown), implying that hgt1Δ/Δ mutants have a markedly reduced expression of a CR3-like protein.

### Reduced binding of recombinant HIV gp160 to C. albicans hgt1Δ/Δ.

Binding of recombinant HIV gp160 to *C. albicans* strains was determined by *Candida* cell ELISA. Human HIV-antibody positive serum followed anti-human-AP IgG revealed a reduced binding of recombinant HIV protein to *C*. *albicans* hgt1Δ/Δ mutants when compared to parental or restored strains (Fig. 6).

### Discussion

Human microbial pathogens, including facultative pathogenic yeasts, are able to counteract the destructive action of complement by acquisition of soluble complement inhibitors FH and C4BP from the host [4,6-8]. We identified a putative FH binding molecule of *C. albicans* by sequencing DNA isolated from clones of a *C. albicans* cDNA expression library, screened with purified FH. The most frequently identified gene was the *C. albicans* HGT1 (high-affinity glucose transporter 1, *CaHGT1*) encoding a putative hexose transporter [15].

In order to evaluate the role and importance of Hgt1p, a homozygous *C. albicans* hgt1Δ/Δ deletion mutant was constructed using a disruption marker cassette and a modified fusion PCR protocol [10]. Although this has not been directly tested, we expect that Hgt2p and other members of this transporter family are able to compensate for the loss of Hgt1 concerning the vital sugar transport, as the deletion mutants are viable, but are likely not factor H binding molecules, as deduced from the marked phenotype of the deletion mutant.

Reduced binding of FH to *C. albicans* hgt1Δ/Δ mutants confirmed that CaHgtlp may indeed be implicated in direct binding of FH. The marked reduction, but not complete lack of binding, may be explained by the presence of other FH-binding proteins in *C. albicans.* Zipfel and coworkers have recently described two FH-binding molecules, the phosphoglycerate mutase (CaGpmlp) [16] and the pH-regulated antigen (Pra1p) [17]. However, Cagmp1 knock-out mutants behaved similar to wild type cells with regard to FH binding [16], and Pralp was not even knocked out as the authors concluded, first, from the finding that only 17% of the FH binding could be inhibited by a Pralp-specific antiserum, and, second, from the existence of different FH binding proteins on the surface of the yeast cells, that a Pra1 knock out mutant will not differ substantially in FH binding [17]. In contrast, the hgt1Δ/Δ mutants used in this study, clearly show a significant decrease in binding (Fig. 3A), implying that CaHgt1p may play a dominant role in the acquisition of FH to the *C. albicans* cell surface.

Microorganisms causing systemic disease employ a variety of complement evasion mechanisms and acquisition of complement regulators is a frequently adopted principle [6]. Interestingly, the same molecule is often employed for binding both the alternative pathway regulator FH and the classical pathway regulator C4BP [6]. We have therefore investigated binding of C4BP and revealed that CaHgtlp appears to be not only a FH-, but also a C4BP-binding molecule. FH ensures that the complement system does not damage endogenous host tissue. However, bound to pathogens, FH may protect them from destruction [6], leading to evasion of host defense by down-regulating complement activation, detected via a reduced formation of the potentially lytic terminal complex (TCC) [6]. Flow cytometry analyses revealed that TCC deposition on the yeast cell surface was much more pronounced when CaHgtlp was absent. Furthermore, in the mutant it was not reduced by preincubation with FH. This confirms that CaHgt1p appears to be a major FH binding molecule and also strongly implies that CaHgt1p plays a functional biological role in downregulating complement activation. This may also be important in the light of the finding that TCC on the fungal surface represents a trigger for the release of C6 and C7 from polymorphonuclear leukocytes (PMNs) [18], indicating that the reduced TCC on the wild type does not trigger PMN functions locally at the site of inflammation. On the other hand, it has been very recently shown that FH bound to the surface of *C. albicans* enhances PMN antimicrobial activity [19], so carrying FH may protect from complement, but likewise enforce neutrophilic attack.

The protein on the PMNs which binds to factor H is the aMb2 integrin CD11b/CD18 or CR3 [19, 20]. As CR3 like molecules have been described for C. *albicans*, reviewed elsewhere [4], it was assessed whether the factor H binding CaHgtlp is such a CR3-like analogue, which are detected via their ability to induce the formation of so called rosettes, i.e. hyphae surrounded by bound complement-coated erythrocytes (EAC, [21]). CR3-like molecules have been shown to be important for fungal virulence and complement evasion in general [22,23], and in particular to be involved in adhesion and filamentous growth [23].

Candida albicans hgt1Δ/Δ mutants fail to form rosettes with complement-coated erythrocytes implying that hgt1Δ/Δ mutants have a markedly reduced expression of a CR3-like protein. Hence, CaHgt1p likely represents a dominant - as no rosettes at all are visible for the deletion mutant - CR3-like protein. A CR3 analogue has previously been identified as a HIV gp160/gp41 binding moiety on *C. albicans* [24]. Upon HIV glycoprotein binding cellular activities augmenting fungal virulence are induced [25], namely enhanced adhesion to HIV infected cells, a decreased phagocytosis of the yeast and an increased Sap release. The reduced binding of HIV-gp160 to the hgt1Δ/Δ mutants indicates that CaHgt1p may also function as a HIV receptor.

The ability to switch between different morphological forms is thought to be an important feature of *C. albicans*, relevant for its pathogenesis and virulence [26]. As the hyphal form is considered to be the more virulent phenotype, the expression library was made from *C*. *albicans* growing in that form. Our results revealing a reduced ability of *C*. *albicans* hgt1Δ/Δ mutants to form hyphae suggest a role of the HGT1 gene for *C. albicans* in modulating hyphae formation. However, this reduced ability did not affect the testing: factor H binding was detected for both hyphal and yeast forms by immunofluorescence (only yeast forms depicted); for FACS experiments yeasts cell have to be taken to avoid a blockage of the nozzle, whereas rosetting and HIV binding studies were done with hyphae. In both latter experiments the incubation was long enough to allow hyphal formation so that the effects are likely not due to an inability of the mutant to generate hyphae, but we cannot fully exclude that some Hgt1 functions are displayed indirectly via an altered cellular morphology as a possibly inferior carbohydrate update may influence the cell wall structure.

It is furthermore interesting that concerning the presence or absence of Hgt1p it is more the quality and not the quantity which matters, so if Hgt1p is present (as in wild type or restored strains) the phenotype is always similar, and for the restored strain not intermediate between normal and mutant. In conclusion, CaHgtlp appears to be a multifunctional complement evasion molecule, causing down-regulation of complement activation by acquisition of FH and C4BP and thus functioning as a complement inhibitor and which may also represent both a CR3 analogue and an HIV binding molecule on Candida. CaHgtlp or derivates may thus be used as prototype for the development of complement inhibitors.

### References

1. Whiteway M, Bachewich C. Morphogenesis in Candida albicans. Ann Rev Microbiol 2007; 61:529-53.
2. Munro CA, Hube B. Anti-fungal therapy at the HAART of viral therapy. Trends Microbiol 2002; 10:177-8.
3. Richardson M, Lass-Florl C. Changing epidemiology of systemic fungal infections. Clin Microbiol Infect 2008; 14:5-24.
4. Speth C, Rambach G, Würzner R, Lass-Florl C. Complement and fungal pathogens: an update. Mycoses 2008; 51:477-96.
5. Józsi M, Zipfel PF. Factor H family proteins and human diseases. Trends Immunol 2008; 29:380-7.
6. Kraiczy P, Würzner R. Complement escape of human pathogenic bacteria by acquisition of complement regulators. Mol Immunol 2006; 43:31-44.
7. Meri T, Hartmann A, Lenk D, Eck R, Würzner R, Hellwage J, Meri S, Zipfel PF. The yeast Candida albicans binds complement regulators factor H and FHL-1. Infect Immun 2002; 70:5185-92.
8. Meri T, Blom AM, Hartmann A, Lenk D, Meri S, Zipfel PF. The hyphal and yeast forms of Candida albicans bind the complement regulator C4b-binding protein. Infect Immun 2004; 72:6633-41.
9. Vogl G, Lesiak I, Jensen DB, Perkhofer S, Eck R, Speth C, Lass-Florl C, Zipfel PF, Blom AM, Dierich MP, Würzner R. Immune evasion by acquisition of complement inhibitors: the mould Aspergillus binds both factor H and C4b binding protein. Mol Immunol 2008; 45:1485-93.
10. Noble SM, Johnson AD. Strains and strategies for large-scale gene deletion studies of the diploid human fungal pathogen Candida albicans. Eucaryotic Cell 2005; 4:298-309.
11. Mathieson PW, Würzner R, Oliveria DB, Lachmann PJ, Peters DK. Complement mediated adipocyte lysis by nephritic factor sera. J Exp Med 1993; 177:1827-31.
12. Hunt SP, Livesey R. Functional genomics. Practical Approach Series 2000; 235:81-112.
13. Sanger, F., S. Nicklen, and A.R. Coulson. 1977. DNA sequencing with chainterminating inhibitors. Proc Nat. Acad Sci U S A 1977; 74:5463-7.
14. Würzner R, Langgartner M, Spbtl L, Eder A, Bujdáková H, Schröppel K, Dierich MP. Temperature-dependent surface expression of the ß2-integrin analogue of Candida albicans and its role in adhesion to the human endothelium. Exp Clin Immunogenet 1996; 13:162-73.
15. Fan J, Chaturvedi V, Shen SH. Identification and phylogenetic analysis of glucose transporter gene family from the human pathogenic yeast Candida albicans. J Mol Evol 2002; 55:336-46.
16. Poltermann S, Kunert A, Von der Heide M, Eck R, Hartmann A, Zipfel PF. Gpmlp is a factor H-, FHL-1-, and plasminogen-binding surface protein of Candida albicans. J Biol Chem 2007; 282:37537-44.
17. Luo S, Poltermann S, Kunert A, Rupp S, Zipfel PF. Immune evasion of the human pathogenic yeast Candida albicans: Pra1 is a Factor H, FHL-1 and plasminogen binding surface protein. Mol Immunol 2009; 47:541-50.
18. Lukasser-Vogl E, Gruber A, Lass-Florl C, Eder A, Hogasen AK, Morgan BP, Dierich MP, Würzner R. Membrane attack complex formation on yeast as trigger of selective release of terminal complement proteins from human polymorphonuclear leukocytes. FEMS Immunol Med Microbiol 2000; 28:15-23.
19. Losse J, Zipfel PF, Józsi M. Factor H and factor H-related protein 1 bind to human neutrophils via complement receptor 3, mediate attachment to Candida albicans, and enhance neutrophil antimicrobial activity. J Immunol 2010; 184:912-21.
20. DiScipio RG, Daffern PJ, Schraufstätter IU, Sriramarao P. Human polymorphonuclear leukocytes adhere to complement factor H through an interaction that involves alphaMbeta2 (CD11b/CD18). J Immunol 1998; 160:4057-66.
21. Heidenreich F, Dierich MP. Candida albicans and Candida stellatoidea, in contrast to other Candida species, bind iC3b and C3d but not C3b. Infect Immun 1985; 50:598-600.
22. Ollert MW, Wadsworth E, Calderone RA. Reduced expression of the functionally active complement receptor for iC3b but not for C3d on an avirulent mutant of Candida albicans. Infect Immun 1990; 59:909-13.
23. Gale CA, Bendel CM, McClellan M, Hauser M, Becker JM. Linkage of adhesion, filamentous growth, and virulence in Candida albicans to a single gene, INT1. Science 1998; 279:1355-1358.
24. Würzner R, Gruber A, Stoiber H, Spruth M, Chen YH, Lukasser-Vogl E, Schwendinger MG, Dierich MP. Human immunodeficiency virus type 1 gp41 binds to Candida albicans via complement C3-like regions. J Infect Dis 1997; 176:492-8.
25. Gruber A, Lukasser-Vogl E, Borg von Zepelin M, Dierich MP, Würzner R. Human immunodeficiency virus type 1 gp160/gp41 binding to Candida albicans selectively enhances candidal virulence in vitro. J Infect Dis 1998; 177:1057-63.
26. Brown AJ, Gow NA. Regulatory networks controlling Candida albicans morphogenesis. Trends Microbiol 1999; 7:333-8.

### Example 2: Production of an antibody against an epitope of CaHgtlp

Polyclonal antibodies directed against a peptide of SEQ ID NO: 6 is raised in rabbits by standard methods. Each animal receives a total amount of 100 µg peptide by repeated subcutaneous injections in monthly intervals. The IgG fraction is purified from the blood taken after 3 months by saturated ammonium sulfate precipitation

## Claims

1. A masking antibody against *Candida albicans* glucose transporter HGT1 (CaHgtlp), wherein the antibody is able to inhibit binding of complement component C3b to CaHgt1p.

2. The masking antibody according to claim 1, wherein the antibody specifically binds to a peptide of the amino acid sequence SEQ ID NO: 6.

3. The masking antibody according to claim 1, wherein the antibody specifically binds to a peptide of the amino acid sequence selected from SEQ ID NO: 4 and SEQ ID NO: 5.

4. The masking antibody according to any one of claims 1 to 3 for use in the treatment of candidiasis.

5. The masking antibody for use in the treatment of candidiasis according to claim 4, wherein the candidiasis is a fungal infection with *Candida albicans.*

6. The masking antibody for use in the treatment of candidiasis according to claim 5, wherein the subject is immunosuppressed or immunocompromised or has a weakened or undeveloped immune system.

7. The masking antibody for use in the treatment of candidiasis according to claims 5 or 6, wherein the subject has or is prone to a metabolic illnesses such as diabetes or has or is prone to cachexia or has or is prone to cancer or has undergone or is undergoing cancer therapy such as chemotherapy or radiotherapy or had or is in need of an organ transplantation.

8. The masking antibody for use in the treatment of candidiasis according to claim 6 or 7, wherein the subject is HIV positive or has AIDS.

## Patentansprüche

1. Maskierender Antikörper gegen den *Candida albicans*-Glucosetransporter HGT1 (CaHgt1p), wobei der Antikörper in der Lage ist, die Bindung der Komplementkomponente C3b an CaHgt1p zu inhibieren.

2. Maskierender Antikörper nach Anspruch 1, wobei der Antikörper spezifisch an ein Peptid der Aminosäuresequenz SEQ ID NO:6 bindet.

3. Maskierender Antikörper nach Anspruch 1, wobei der Antikörper spezifisch an ein Peptid der aus SEQ ID NO:4 und SEQ ID NO:5 ausgewählten Aminosäuresequenz bindet.

4. Maskierender Antikörper nach einem der Ansprüche 1 bis 3 zur Verwendung bei der Behandlung von Candidose.

5. Maskierender Antikörper zur Behandlung von Candidose nach Anspruch 4, wobei die Candidose eine Pilzinfektion mit *Candida albicans* ist.

6. Maskierender Antikörper zur Verwendung bei der Behandlung von Candidose nach Anspruch 5, wobei das Individuum immunsupprimiert oder immunkompromittiert ist oder ein geschwächtes oder nicht entwickeltes Immunsystem hat.

7. Maskierender Antikörper zur Verwendung bei der Behandlung von Candidose nach Anspruch 5 oder 6, wobei das Individuum an einer Stoffwechselerkrankung wie Diabetes leidet oder dafür anfällig ist oder unter Kachexie leidet oder dafür anfällig ist oder Krebs hat oder dafür anfällig ist oder sich einer Krebstherapie wie Chemotherapie oder Strahlentherapie unterzogen hat bzw. sich einer solchen Therapie unterzieht oder eine Organtransplantation hatte oder einer solchen bedarf.

8. Maskierender Antikörper zur Verwendung bei der Behandlung von Candidose nach Anspruch 6 oder 7, wobei das Individuum HIV-positiv ist oder AIDS hat.

## Revendications

1. Anticorps de masquage contre le transporteur de glucose de *Candida albicans* HGT1 (CaHgt1p.), l'anticorps étant capable d'inhiber la liaison du composant du complément C3b à CaHgt1p.

2. Anticorps de masquage selon la revendication 1, l'anticorps se liant spécifiquement à un peptide ayant la séquence d'acides aminés SEQ ID NO:6.

3. Anticorps de masquage selon la revendication 1, l'anticorps se liant spécifiquement à un peptide ayant la séquence d'acides aminés choisie parmi SEQ ID NO:4 et SEQ ID NO:5.

4. Anticorps de masquage selon l'une quelconque des revendications 1 à 3, pour une utilisation dans le traitement de la candidose.

5. Anticorps de masquage pour une utilisation dans le traitement de la candidose selon la revendication 4, la candidose étant une infection fongique à *Candida albicans.*

6. Anticorps de masquage pour une utilisation dans le traitement de la candidose selon la revendication 5, le sujet étant immunosupprimé ou immunocompromis ou ayant un système immunitaire affaibli ou non-développé.

7. Anticorps de masquage pour une utilisation dans le traitement de la candidose selon les revendications 5 ou 6, le sujet présentant une maladie métabolique telle que le diabète, ou y étant prédisposé, ou présentant une cachexie ou y étant prédisposé, ou présentant un cancer ou y étant prédisposé, ou ayant subi ou subissant un traitement du cancer tel qu'une chimiothérapie ou une radiothérapie, ou ayant eu une transplantation d'organe ou nécessitant celle-ci.

8. Anticorps de masquage pour une utilisation dans le traitement de la candidose selon la revendication 6 ou 7, le sujet étant VIH-positif et présentant un SIDA.


### SEQUENCE LISTING

<110> Medizinische UniversitÃ¤t Innsbruck
<120> CaHgt1p inhibitors for use in the treatment of Candidiasis
<130> T2302 PCT S3
<160> 9
<170> BiSSAP 1.0
<210> 1
   <211> 3288
   <212> DNA
   <213> Candida albicans
<220>
   <221> source
   <222> 1..3288
   <223> /mol_type="DNA" /organism="Candida albicans"
<400> 1
   gcagacagca attcatttgg agagtggcaa gtctacacga atttagctaa ggttcactat 60
   atcgtgtaac aagaaatttc tataccaaat aaacagcact tgattgaact acgatatgta 120
   aaaacttgct tttattacca gtcttcatac ataccccggt cttctctttt caatattctg 180
   tatatgtctt tacaactctt aacactccgt aaatgtgcct ttcgaatact tttgcagctg 240
   gatatttttc cggtgcacct tttcagttat cttttgcaac ttttcgcgag caatgacaaa 300
   agtttggggc gtgaggcaac aaaatgcatg gcattaccag tacagtatcg ccacaagtgg 360
   ttttccttgg catttcttga ttgtttagta gaacaattca ataagacttt tttgatcatg 420
   aatttttttt gccatgaagg tgctttcatt gttcaaggtt gaaggggaat tgaaaaattt 480
   gtagagtcac aatcaaatgg cttgataatt tgataaaaaa aaaaagaacc cttaaaaaat 540
   attcatacca atgtatgcat aaccataaag aacttaataa ttatgcacct gcaatcagaa 600
   agtcatttct tacgatgatt tgccaaatga ccgtaaaacg actagcaaaa acagtgacat 660
   ttttttttga aaaggtggag atgaaaacca ttctggtttg tttcgtcatt tacacaaata 720
   ttcgacacaa aaactattaa ttcaatacaa acaaaaaaat gtgcaggaag tcttggaacc 780
   gatacaaaaa tttttacaaa ccatgtacac tgttgttttg gggaagaatt agtcggggaa 840
   gaaggcccag aaacttgagt aaagagtgga ttcaacactt tataatagta tcattttgta 900
   acacaaaaat gaaatacacc caataaaaac tgttgaaaca tttatccgtc aagcttattc 960
   gatggagtac aacactttac atttcttccg aaacaataac tatataaacc catgtaagtc 1020
   tcccctcttt tgtttcaaac gtcttaccaa tttttctctt cactactttt ccaacttaac 1080
   aatcttcact tataatctca acgaatcatg tcgtccaaga tcgaaagaat cttttctgga 1140
   cctgctttaa aaatcaatac ttatttggac aagcttccaa aaatttacaa tgttttcttc 1200
   atcgctagta tatccaccat tgctggtatg atgtttggtt ttgatatttc ttcaatgtct     1260
   gcctttattg gtgctgaaca ttatatgagg tatttcaatt ccccaggatc ggatatccaa     1320
   gggttcatta cttcatccat ggctttgggt tctttctttg gttctattgc ctcctcattt     1380
   gtttctgaac cctttggtag aagattatca ttattgactt gtgctttctt ctggatggtc     1440
   ggtgctgcta ttcaatcatc agttcaaaat cgtgctcaat tgattattgg tagaatcatt     1500
   tctggtattg gtgttggttt cggttctgct gttgctccag tttatggtgc tgaattggct     1560
   ccaagaaaaa ttagaggtct tatcggtggt atgttccaat tctttgtcac cttgggtatc     1620
   atgatcatgt tttacttatc cttcggattg ggccacatta atggtgttgc ttcattcaga     1680
   attgcttggg gacttcaaat tgtccctggt ctttgtttgt tcttgggttg tttctttatt     1740
   ccagaatctc ctcgttggtt ggctaaacaa ggtcaatggg aagctgctga agaaattgtt     1800
   gccaagatcc aagctcatgg tgatcgtgaa aatcctgatg ttttgattga aatttctgaa     1860
   atcaaagatc aattattatt ggaggagtcc agtaaacaaa ttggatatgc tactttattc     1920
   accaaaaaat acattcaaag aaccttcact gctattttcg ctcaaatttg gcaacaattg     1980
   actggtatga acgttatgat gtactatatc gtttatattt tccaaatggc tggttattct     2040
   ggtaacagta acttggttgc ttcatccatt caatatgtta tcaacacttg tgtcaccgtg     2100
   cctgcattat attttattga taaagtaggt agaagaccat tattgattgg tggtgccact     2160
   atgatgatgg ctttccaatt cggtcttgct ggtattttgg gtcaatactc aattccttgg     2220
   ccagatagtg gtaacgactc agtcaacatt agaattccag aagacaataa gtctgcctcc     2280
   aaaggtgcca ttgcttgttg ctacttgttc gttgcttcct ttgcctttac ttggggggtc      2340
   ggtatttggg tctactgtgc tgaaatatgg ggtgataaca gagttgctca acgtggtaat     2400
   gccatctcca cttctgccaa ttggattttg aatttcgcta ttgccatgta taccccaact     2460
   ggtttcaaga atattagctg gaaaacatat atcatttatg gtgtcttctg tttcgctatg     2520
   gctacccatg tctactttgg attcccagaa accaagggta aaagattgga agaaattggt     2580
   caaatgtggg aagaacgtgt tcctgcttgg agatcaagat cttggcaacc aaccgttcct     2640
   attgcttccg atgccgaatt ggcaagaaag atggaagttg aacacgaaga agataaattg     2700
   atgaatgaag actctaactc cgaatccaga gaaaatcaag cctaaatgga atttagttgc     2760
   aacttcaatt cttcattttt tcaaattcat tatttcaaaa tacctattct gttgttttca     2820
   aatgaaaaat tagtataatg tttctttaat tcgttatgtg tgtagatatt tgttattaaa     2880
   agaataaatt cttttttatc agtattgttt tcgttagtcc tacttgctga agattaaatt      2940
   ttttttctga agcagtttcg tcaaaaagtt gtggtaaaga tatttacctc caacagctta      3000
   tttagacaga attgacaagt catataataa atcagtattt gtcacttcaa cctgtgcacg     3060
   tatgcgtctg ccacaatgaa catttgtcaa ttgttaaaaa tgcaaaccaa gtagttctaa 3120
   acagctattc attattgttg aatggctgca ataacgttta ttcaatcatc aagcaaaata 3180
   gtaaccagtt caagctagtt acttttgaag actatcacta cgcaatagtt actcgacaac 3240
   caatatttaa caatacactt aaagggaaag tccacaacaa cactaaac 3288
<210> 2
   <211> 545
   <212> PRT
   <213> Candida albicans
<220>
   <221> SOURCE
   <222> 1..545
   <223> /mol_type="protein" /organism="Candida albicans"
<400> 2
   Met Ser Ser Lys Ile Glu Arg Ile Phe Ser Gly Pro Ala Leu Lys Ile
   1               5                   10                  15
   Asn Thr Tyr Leu Asp Lys Leu Pro Lys Ile Tyr Asn Val Phe Phe Ile
               20                  25                  30
   Ala Ser Ile Ser Thr Ile Ala Gly Met Met Phe Gly Phe Asp Ile Ser
           35                  40                  45
   Ser Met Ser Ala Phe Ile Gly Ala Glu His Tyr Met Arg Tyr Phe Asn
       50                  55                  60
   Ser Pro Gly Ser Asp Ile Gln Gly Phe Ile Thr Ser Ser Met Ala Leu
   65                  70                  75                  80
   Gly Ser Phe Phe Gly Ser Ile Ala Ser Ser Phe Val Ser Glu Pro Phe
                   85                  90                  95
   Gly Arg Arg Leu Ser Leu Leu Thr Cys Ala Phe Phe Trp Met Val Gly
               100                 105                 110
   Ala Ala Ile Gln Ser Ser Val Gln Asn Arg Ala Gln Leu Ile Ile Gly
           115                 120                 125
   Arg Ile Ile Ser Gly Ile Gly Val Gly Phe Gly Ser Ala Val Ala Pro
       130                 135                 140
   Val Tyr Gly Ala Glu Leu Ala Pro Arg Lys Ile Arg Gly Leu Ile Gly
   145                 150                 155                 160
   Gly Met Phe Gln Phe Phe Val Thr Leu Gly Ile Met Ile Met Phe Tyr
                   165                 170                 175
   Leu Ser Phe Gly Leu Gly His Ile Asn Gly Val Ala Ser Phe Arg Ile
               180                 185                 190
   Ala Trp Gly Leu Gln Ile Val Pro Gly Leu Cys Leu Phe Leu Gly Cys
           195                 200                 205
   Phe Phe Ile Pro Glu Ser Pro Arg Trp Leu Ala Lys Gln Gly Gln Trp
       210                 215                 220
   Glu Ala Ala Glu Glu Ile Val Ala Lys Ile Gln Ala His Gly Asp Arg
   225                 230                 235                 240
   Glu Asn Pro Asp Val Leu Ile Glu Ile Ser Glu Ile Lys Asp Gln Leu
                   245                 250                 255
   Leu Leu Glu Glu Ser Ser Lys Gln Ile Gly Tyr Ala Thr Leu Phe Thr
               260                 265                 270
   Lys Lys Tyr Ile Gln Arg Thr Phe Thr Ala Ile Phe Ala Gln Ile Trp
           275                 280                 285
   Gln Gln Leu Thr Gly Met Asn Val Met Met Tyr Tyr Ile Val Tyr Ile
       290                 295                 300
   Phe Gln Met Ala Gly Tyr Ser Gly Asn Ser Asn Leu Val Ala Ser Ser
   305                 310                 315                 320
   Ile Gln Tyr Val Ile Asn Thr Cys Val Thr Val Pro Ala Leu Tyr Phe
                   325                 330                 335
   Ile Asp Lys Val Gly Arg Arg Pro Leu Leu Ile Gly Gly Ala Thr Met
               340                 345                 350
   Met Met Ala Phe Gln Phe Gly Leu Ala Gly Ile Leu Gly Gln Tyr Ser
           355                 360                 365
   Ile Pro Trp Pro Asp Ser Gly Asn Asp Ser Val Asn Ile Arg Ile Pro
       370                 375                 380
   Glu Asp Asn Lys Ser Ala Ser Lys Gly Ala Ile Ala Cys Cys Tyr Leu
   385                 390                 395                 400
   Phe Val Ala Ser Phe Ala Phe Thr Trp Gly Val Gly Ile Trp Val Tyr
                   405                 410                 415
   Cys Ala Glu Ile Trp Gly Asp Asn Arg Val Ala Gln Arg Gly Asn Ala
               420                 425                 430
   Ile Ser Thr Ser Ala Asn Trp Ile Leu Asn Phe Ala Ile Ala Met Tyr
           435                 440                 445
   Thr Pro Thr Gly Phe Lys Asn Ile Ser Trp Lys Thr Tyr Ile Ile Tyr
       450                 455                 460
   Gly Val Phe Cys Phe Ala Met Ala Thr His Val Tyr Phe Gly Phe Pro
   465                 470                 475                 480
   Glu Thr Lys Gly Lys Arg Leu Glu Glu Ile Gly Gln Met Trp Glu Glu
                   485                 490                 495
   Arg Val Pro Ala Trp Arg Ser Arg Ser Trp Gln Pro Thr Val Pro Ile
               500                 505                 510
   Ala Ser Asp Ala Glu Leu Ala Arg Lys Met Glu Val Glu His Glu Glu
           515                 520                 525
   Asp Lys Leu Met Asn Glu Asp Ser Asn Ser Glu Ser Arg Glu Asn Gln
       530                 535                 540
   Ala
   545
<210> 3
   <211> 544
   <212> PRT
   <213> Candida albicans
<220>
   <221> SOURCE
   <222> 1..544
   <223> /mol_type="protein" /organism="Candida albicans"
<400> 3
   Met Ser Ser Lys Ile Glu Arg Ile Phe Ser Gly Pro Ala Leu Lys Ile
   1               5                   10                  15
   Asn Thr Tyr Leu Asp Lys Leu Pro Lys Ile Tyr Asn Val Phe Phe Ile
               20                  25                  30
   Ala Ser Ile Ser Thr Ile Ala Gly Met Met Phe Gly Phe Asp Ile Ser
           35                  40                  45
   Ser Met Ser Ala Phe Ile Gly Ala Glu His Tyr Met Arg Tyr Phe Asn
       50                  55                  60
   Ser Pro Gly Ser Asp Ile Gln Gly Phe Ile Thr Ser Ser Met Ala Leu
   65                  70                  75                  80
   Gly Ser Phe Phe Gly Ser Ile Ala Ser Ser Phe Val Ser Glu Pro Phe
                   85                  90                  95
   Gly Arg Arg Leu Ser Leu Leu Thr Cys Ala Phe Phe Trp Met Val Gly
               100                 105                 110
   Ala Ala Ile Gln Ser Ser Val Gln Asn Arg Ala Gln Leu Ile Ile Gly
           115                 120                 125
   Arg Ile Ile Ser Gly Ile Gly Val Gly Phe Gly Ser Ala Val Ala Pro
       130                 135                 140
   Val Tyr Gly Ala Glu Leu Ala Pro Arg Lys Ile Arg Gly Leu Ile Gly
   145                 150                 155                 160
   Gly Met Phe Gln Phe Phe Val Thr Leu Gly Ile Met Ile Met Phe Tyr
                   165                 170                 175
   Leu Ser Phe Gly Leu Gly His Ile Asn Gly Val Ala Ser Phe Arg Ile
               180                 185                 190
   Ala Trp Gly Leu Gln Ile Val Pro Gly Leu Cys Leu Phe Leu Gly Cys
           195                 200                 205
   Phe Phe Ile Pro Glu Ser Pro Arg Trp Leu Ala Lys Gln Gly Gln Trp
       210                 215                 220
   Glu Ala Ala Glu Glu Ile Val Ala Lys Ile Gln Ala His Gly Asp Arg
   225                 230                 235                 240
   Glu Asn Pro Asp Val Leu Ile Glu Ile Ser Glu Ile Lys Asp Gln Leu
                   245                 250                 255
   Leu Leu Glu Glu Ser Ser Lys Gln Ile Gly Tyr Ala Thr Leu Phe Thr
               260                 265                 270
   Lys Lys Tyr Ile Gln Arg Thr Phe Thr Ala Ile Phe Ala Gln Ile Trp
           275                 280                 285
   Gln Gln Leu Thr Gly Met Asn Val Met Met Tyr Tyr Ile Val Tyr Ile
       290                 295                 300
   Phe Gln Met Ala Gly Tyr Ser Gly Asn Ser Asn Leu Val Ala Ser Ser
   305                 310                 315                 320
   Ile Gln Tyr Val Ile Asn Thr Cys Val Thr Val Pro Ala Leu Tyr Phe
                   325                 330                 335
   Ile Asp Lys Val Gly Arg Arg Pro Leu Leu Ile Gly Gly Ala Thr Met
               340                 345                 350
   Met Met Ala Phe Gln Phe Gly Leu Ala Gly Ile Leu Gly Gln Tyr Ser
           355                 360                 365
   Ile Pro Trp Pro Asp Ser Gly Asn Asp Ser Val Asn Ile Arg Ile Pro
       370                 375                 380
   Glu Asp Asn Lys Ser Ala Ser Lys Gly Ala Ile Ala Cys Cys Tyr Leu
   385                 390                 395                 400
   Phe Val Ala Ser Phe Ala Phe Thr Trp Gly Val Gly Ile Trp Val Tyr
                   405                 410                 415
   Cys Ala Glu Ile Trp Gly Asp Asn Arg Val Ala Gln Arg Gly Asn Ala
               420                 425                 430
   Ile Ser Thr Ser Ala Asn Trp Ile Leu Asn Phe Ala Ile Ala Met Tyr
           435                 440                 445
   Thr Pro Ser Gly Phe Lys Asn Ile Ser Trp Lys Thr Tyr Ile Ile Tyr
       450                 455                 460
   Gly Val Phe Cys Phe Ala Met Ala Thr His Val Tyr Phe Gly Phe Pro
   465                 470                 475                 480
   Glu Thr Lys Gly Lys Arg Leu Glu Glu Ile Gly Gln Met Trp Glu Glu
                   485                 490                 495
   Arg Val Pro Ala Trp Arg Ser Arg Ser Trp Gln Pro Thr Val Pro Ile
               500                 505                 510
   Ala Ser Asp Ala Glu Leu Ala Arg Lys Met Glu Val Glu His Glu Glu
           515                 520                 525
   Asp Lys Leu Met Asn Glu Asp Ser Asn Ser Glu Ser Arg Glu Asn Gln
       530                 535                 540
<210> 4
   <211> 27
   <212> PRT
   <213> Candida albicans
<220>
   <221> SOURCE
   <222> 1..27
   <223> /mol_type="protein" /organism="Candida albicans"
<400> 4
   Phe Asp Ile Ser Ser Met Ser Ala Phe Ile Gly Ala Glu His Tyr Met
   1               5                   10                  15
   Arg Tyr Phe Asn Ser Pro Gly Ser Asp Ile Gln
               20                  25
<210> 5
   <211> 20
   <212> PRT
   <213> Candida albicans
<220>
   <221> SOURCE
   <222> 1..20
   <223> /mol_type="protein" /organism="Candida albicans"
<400> 5
   Gln Tyr Ser Ile Pro Trp Pro Asp Ser Gly Asn Asp Ser Val Asn Ile
   1               5                   10                  15
   Arg Ile Pro Glu
               20
<210> 6
   <211> 9
   <212> PRT
   <213> Candida albicans
<220>
   <221> SOURCE
   <222> 1..9
   <223> /mol_type="protein" /organism="Candida albicans"
<400> 6
   Ala Glu His Tyr Met Arg Tyr Phe Asn
   1               5
<210> 7
   <211> 16
   <212> PRT
   <213> Candida albicans
<220>
   <221> SOURCE
   <222> 1..16
   <223> /mol_type="protein" /organism="Candida albicans"
<400> 7
   Ile Val Tyr Ile Phe Gln Met Ala Gly Tyr Ser Gly Asn Ser Asn Leu
   1               5                   10                  15
<210> 8
   <211> 11
   <212> PRT
   <213> Candida albicans
<220>
   <221> SOURCE
   <222> 1..11
   <223> /mol_type="protein" /organism="Candida albicans"
<400> 8
   Leu Gly His Ile Asn Gly Val Ala Ser Phe Arg
   1               5                   10
<210> 9
   <211> 9
   <212> PRT
   <213> Candida dubliniensis
<220>
   <221> SOURCE
   <222> 1..9
   <223> /mol_type="protein" /organism="Candida dubliniensis"
<400> 9
   Ala Asp His Tyr Met Arg Tyr Phe Asn
   1               5